# EUROPEAN PATENT SPECIFICATION

(11) **EP 1 639 014 B1**
(45) Date of publication and mention of the grant of the patent: **22.09.2010**
(21) Application number: 04776502.9
(22) Date of filing: 14.06.2004
(51) Int. Cl.: C07K 16/28, A61K 39/395, C12N 5/10

(54) **AGLYCOSYL ANTI-CD154 (CD40 LIGAND) ANTIBODIES AND USES THEREOF**
AGLYCOSYL-ANTI-CD154 (CD40-LIGAND) ANTIKÖRPER UND DEREN VERWENDUNGEN
ANTICORPS D'AGLYCOSYLE ANTI-CD154 (LIGAND CD40) ET UTILISATIONS CORRESPONDANTES

(30) Priority: 13.06.2003 US 478284 P; 24.07.2003 US 490186 P
(43) Date of publication of application: 29.03.2006
(60) Divisional application: 10155543.1
(73) Proprietor: Biogen Idec MA Inc., Cambridge, Massachusetts 02142 (US)
(72) Inventor: TAYLOR, Frederick, R., Milton, MA 02186 (US); BENJAMIN, Christopher, D., Beverly, MA 01915 (US); BURKLY, Linda, C., West Newton, MA 02465 (US); GARBER, Ellen, A., Cambridge, MA 02138-1352 (US)
(74) Representative: Adams, Harvey Vaughan John
(86) International application number: PCT/US2004/018708
(87) International publication number: WO 2005/003175

(56) References cited:
- WO-A-01/79555
- WO-A-01/93908
- WO-A-93/19196
- WO-A-98/58672
- WO-A-03/101485
- US-A- 5 474 771
- US-B1- 6 312 692
- FRIEND P J ET AL: "PHASE I STUDY OF AN ENGINEERED AGLYCOSYLATED HUMANIZED CD3 ANTIBODY IN RENAL TRANSPLANT REJECTION" TRANSPLANTATION, WILLIAMS AND WILKINS, BALTIMORE, MD, US, vol. 68, no. 11, 15 December 1999 (1999-12-15), pages 1632-1637, XP009011271 ISSN: 0041-1337
- ISAACS J D ET AL: "THERAPY WITH MONOCLONAL ANTIBODIES AN IN-VIVO MODEL FOR THE ASSESSMENT OF THERAPEUTIC POTENTIAL" JOURNAL OF IMMUNOLOGY, vol. 148, no. 10, 1992, pages 3062-3071, XP002310165 ISSN: 0022-1767
- BURKLY L C: "CD40 pathway blockade as an approach to immunotherapy." 2001, ADVANCES IN EXPERIMENTAL MEDICINE AND BIOLOGY. 2001, VOL. 489, PAGE(S) 135 - 152 , XP009041482 ISSN: 0065-2598 the whole document

## Description

### Technical Field of the Invention

The present invention relates to aglycosyl anti-CD154 antibodies or antibody derivatives thereof, which block the interaction of CD154 and CD40 molecules. Methods for producing the aglycosyl anti-CD154 antibodies and antibody derivatives are disclosed herein. The antibodies and antibody derivatives of the present invention are useful in the treatment and prevention of diseases that involve undesirable immune responses, and that are mediated by CD154-CD40 interactions.

### Background Of The Invention

The generation of humoral and cell-mediated immunity is orchestrated by the interaction of activated helper T cells with antigen-presenting cells ("APCs") and effector T cells. Activation of the helper T cells is not only dependent on the interaction of the antigen-specific T-cell receptor ("TCR") with its cognate peptide-MHC ligand, but also requires the coordinate binding and activation by a number of cell adhesion and costimulatory molecules [Salazar-Fontana, 2001].

A critical costimulatory molecule is CD154 (also known as CD40 ligand, CD40L, gp39, T-BAM, T-Cell Activating Molecule, TRAP), a Type II transmembrane protein that is expressed in an activation-dependent, temporally-restricted, manner on the surface of CD4⁺ T cells. CD154 is also expressed, following activation, on a subset of CD8⁺ T cells, basophils, mast cells, eosinophils, natural killer cells, B cells, macrophages, dendritic cells and platelets.

The CD154 counter-receptor, CD40, is a Type I membrane protein that is constitutively and widely expressed on the surface of many cell types, including APCs [Foy, 1996].

Signaling through CD40 by CD154 initiates a cascade of events that result in the activation of the CD40 receptor-bearing cells and optimal CD4⁺ T cell priming. More specifically, the cognate interaction between CD154 and CD40 promotes the differentiation of B cells into antibody secreting cells and memory B cells [Burkly, 2001]. Additionally, the CD154-CD40 interaction promotes cell-mediated immunity through the activation of macrophages and dendritic cells and the generation of natural killer cells and cytotoxic T lymphocytes [Burkly, 2001].

The pivotal role of CD154 in regulating the function of both the humoral and cell-mediated immune response has provoked great interest in the use of inhibitors of this pathway for therapeutic immunomodulation [United States patent 5,474,771]. As such, anti-CD154 antibodies have been shown to be beneficial in a wide variety of models of immune response to other therapeutic proteins or gene therapy, allergens, autoimmunity and transplantation [United States patent 5,474,771; Burkly, 2001].

The CD40-CD154 interaction has been shown to be important in several experimentally induced autoimmune diseases, such as collagen-induced arthritis, experimental allergic encephalomyelitis ("EAE"), oophoritis, colitis, and drug-induced lupus nephritis. Specifically, it has been shown that disease induction in all of these models can be blocked with CD154 antagonists at the time of antigen administration [Burkly, 2001].

The blockade of disease using anti-CD154 antagonists has also been seen in animal models of spontaneous autoimmune disease, including insulin-dependent diabetes and lupus nephritis, as well as in graft-vs-host disease, transplant, pulmonary fibrosis, and atherosclerosis disease models [Burkly, 2001].

Although glycosylated anti-CD154 antibodies have proven useful for the prevention and treatment of several immune response-related diseases, in some subjects, therapies using them are sometimes complicated by thromboembolitic activity [Biogen Press Release, 2001; IDEC Press Release, 2001]. Although the mechanism of this side effect is unknown, it could involve the colligation by the anti-CD154 antibody, or aggregates thereof, of FcγRIIa and CD154 on platelets, leading to inappropriate platelet activation. Binding to other Fcγ receptors and complement could also potentiate this effect. Thus, forms of anti-CD154 antibodies that do not bind to effector receptors may be safer and/or more effective for therapeutic use.

The mechanism by which anti-CD154 antibodies inhibit immune function may be more complex than simple binding to CD154 to block interactions with CD40 and, in fact, may include contributions by effector pathways. For example, antibody-antigen binding may induce deletion of activated T cells through Fc domain binding to Fcγ receptors or complement components. Alternatively, binding of the antibody to CD154 may be enhanced by the formation of a cell surface scaffold of the antibody on Fcγ receptor-bearing cells. In addition, access of the antibody to its site of action may be promoted by Fcγ receptor binding interactions.

In glycosylated antibodies, including anti-CD154 antibodies, the glycans attached to the conserved N-linked site in the C_{H2} domains of the Fc dimer are enclosed between the C_{H2} domains, with the sugar residues making contact with specific amino acid residues on the opposing C_{H2} domain [Jeffries, 1998]. *In vitro* studies with various glycosylated antibodies have demonstrated that removal of the C_{H2} glycans alters the Fc structure such that antibody binding to Fc receptors and the complement protein C1Q are greatly reduced [Nose, 1983; Leatherbarrow, 1985; Tao, 1989; Lund, 1990; Dorai, 1991; Hand, 1992; Leader, 1991; Pound, 1993; Boyd, 1995]. *In vivo* studies have confirmed the reduction in the effector function of aglycosyl antibodies. For example, an aglycosyl anti-CD8 antibody is incapable of depleting CD8-bearing cells in mice [Isaacs, 1992] and an aglycosyl anti-CD3 antibody does not induce cytokine release syndrome in mice or humans [Boyd, 1995; Friend, 1999].

While removal of the glycans in the C_{H2} domain appears to have a significant effect on effector function, other functional and physical properties of the antibody remain unaltered. Specifically, it has been shown that removal of the glycans had little to no effect on serum half-life and binding to antigen [Nose, 1983; Tao, 1989; Dorai, 1991; Hand, 1992; Hobbs, 1992].

### Summary Of The Invention

In accordance with this invention, the Fc effector function involved in the mechanism of action of anti-CD154 antibodies is elucidated through the use of an anti-CD154 antibody in which Fc effector function has been reduced by a modification of the conserved N-linked site in the C_{H2} domains of the Fc dimer, leading to "aglycosyl" anti-CD154 antibodies. Examples of such modifications include mutation of the conserved N-linked site in the C_{H2} domains of the Fc dimer, removal of glycans attached to the N-linked site in the C_{H2} domains and prevention of glycosylation.
To address whether the mechanism of inhibition by anti-CD154 antibody depends on its Fc effector interactions, anti-CD154 antibody and its aglycosyl counterpart were compared with regard to their ability to inhibit several diseases via blocking the CD154-CD40 interaction. The results reported herein demonstrate that aglycosylated forms of the anti-CD154 antibody are equally protective as the glycosylated forms of the anti-CD154 antibody.

Because the aglycosyl anti-CD154 antibodies of this invention are characterized by diminished effector function, these antibodies are particularly desirable for use in subjects where the potential for undesirable thromboembolitic activity exists. Additionally, the diminished Fc effector function of the aglycosyl anti-CD154 antibodies may decrease or eliminate other potential side effects of anti-CD154 antibody therapies, such as deletion of activated T cells and other populations of cells induced to express CD154 or Fc-dependant activation of monocytes/macrophages.

Specifically, this invention provides aglycosyl anti-CD154 antibodies that recognize CD154. More particularly, this invention provides a humanized, aglycosylated anti-CD154 antibody -- namely "aglycosyl hu5c8", and a murine, aglycosylated anti-CD154 antibody -- namely "aglycosyl muMR1".

In one embodiment of this invention, the aglycosyl hu5c8 antibody is produced from the NS0 aglycosyl hu5c8 cell line, which was deposited with the American Type Culture Collection ("ATCC"), 10801 University Blvd., Manassas, Virginia on January 14, 2003 (Accession No. PTA-4931), and the aglycosyl MR1 antibody is produced from the NS0 aglycosyl murine MR1 cell line that was deposited with the ATCC on January 14, 2003 (Accession No. PTA-4934).

In one embodiment of this invention, aglycosyl anti-CD154 antibodies are capable of inhibiting the interaction between CD154 and CD40.

In another embodiment of this invention, aglycosyl anti-CD154 antibodies are able to associate with CD154 in a manner that blocks, directly or indirectly the activation of CD40-bearing cells.

Also disclosed herein is a method of inhibiting an immune response in a subject, comprising administering to the subject an aglycosyl anti-CD154 antibody or an antibody derivative thereof, wherein the antibody or antibody derivative is administered in an amount effective to inhibit activation of the immune cells in the subject.

Also disclosed herein is a method of treating or preventing, in a subject, an immune response-dependent condition or disease, comprising administering to the subject an aglycosyl anti-CD154 antibody or an antibody derivative thereof, the antibody or antibody derivative being administered in an amount effective to inhibit activation of the immune cells in the subject and thereby treat or prevent the immune response-dependent condition or disease.

### Brief Description Of The Drawings

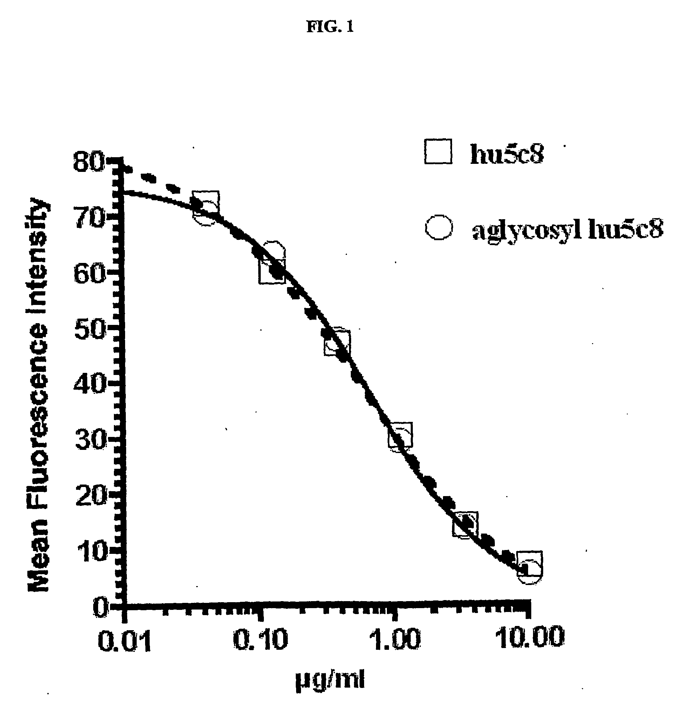

FIG. 1 illustrates that aglycosyl hu5c8 monoclonal antibody ("mAb") and glycosylated hu5c8 mAb bind to human CD154 with the same relative affinity. The binding of biotinylated hu5c8 mAb to cell surface CD154 was competed with titrations of unlabeled glycosylated hu5c8 mAb or aglycosyl hu5c8 mAb. The mean fluorescence intensity of the biotinylated antibody detected with streptavidin-PE was plotted versus the concentration of unlabeled antibody. Four parameter curve fits are depicted, collectively.

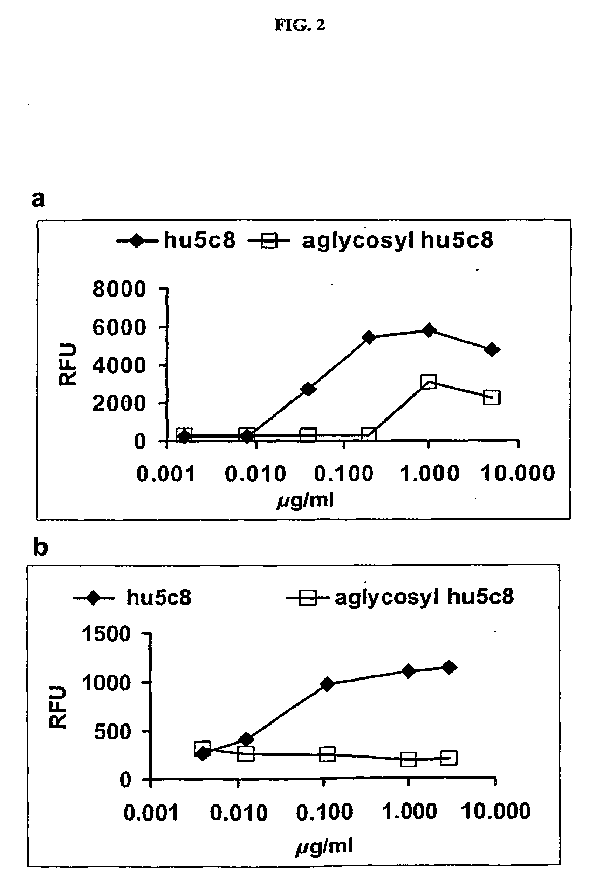

FIG. 2 illustrates that aglycosyl hu5c8 mAb has impaired FcR binding capabilities. The ability of an anti-CD154 antibody, namely aglycosyl hu5c8 mAb, to form a bridge between huCD154 and FcγRI⁺ cells (a) or between huCD154⁺ CHO cells and FcγRIII⁺ cells (b) was evaluated. Fluorescently labeled FcγR⁺ cells were added to microtiter plates containing glycosylated hu5c8 mAb or aglycosyl hu5c8 mAb that was prebound to CD154. Bound FcγR⁺ cells were detected by measuring the relative fluorescent units ("RFU") in each well using the excitation/emission spectra, 485/530 nm.

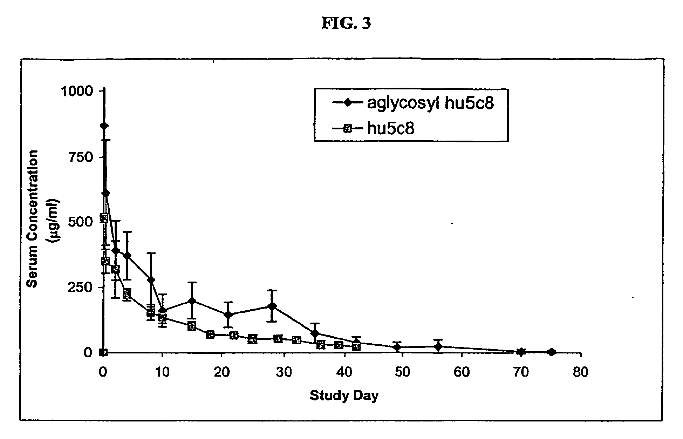

FIG. 3 illustrates that glycosylated hu5c8 mAb and aglycosyl hu5c8 mAb have the same serum half-life in cynomolgus monkeys. The concentrations of glycosylated hu5c8 mAb and aglycosyl hu5c8 mAb were measured in the serum of cynomolgus monkeys after the administration of a single 20mg/kg intravenous dose. The mean serum concentrations for each treatment group are depicted ± standard deviation ("SD").

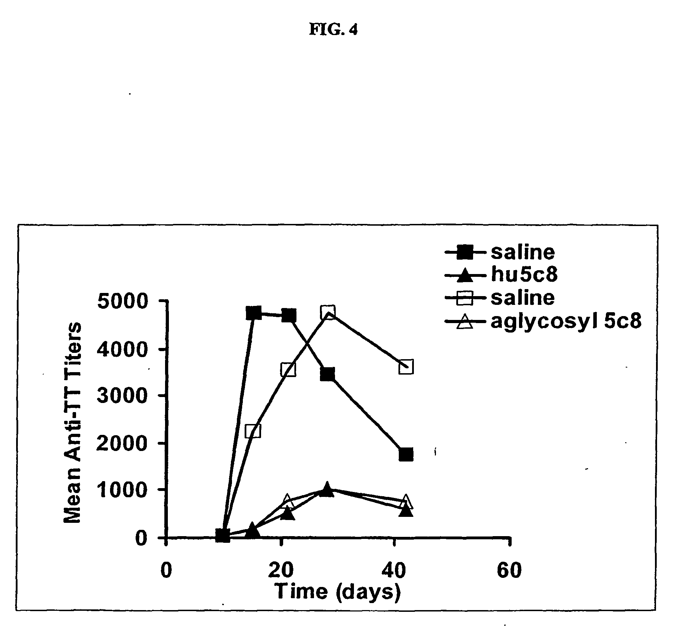

FIG. 4 illustrates that glycosylated hu5c8 mAb and aglycosyl hu5c8 mAb inhibit a primary immune response to tetanus toxoid ("TT"). Results for cynomolgus monkeys in the mAb treated and saline control groups in the glycosylated hu5c8 mAb study (closed symbols) and in the aglycosyl hu5c8 mAb (open symbols) are depicted.

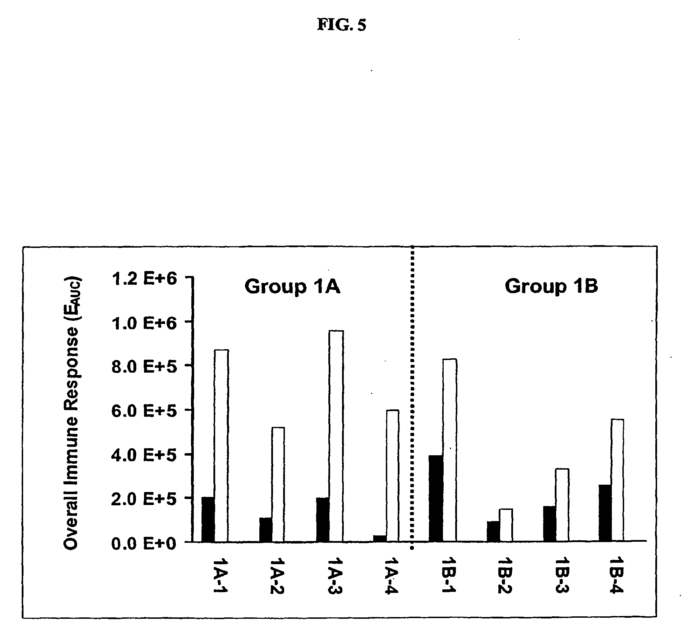

FIG. 5 illustrates that aglycosyl hu5c8 mAb inhibits a secondary immune response to TT. Primary (closed bars) and secondary (open bars) overall antibody responses (E_{AUC}) to TT for individual cynomolgus monkeys are depicted. Group 1A animals received saline prior to both the primary and secondary TT challenges. Group 1B animals received saline prior to the primary TT challenge and aglycosyl hu5c8 mAb prior to the secondary TT challenge.

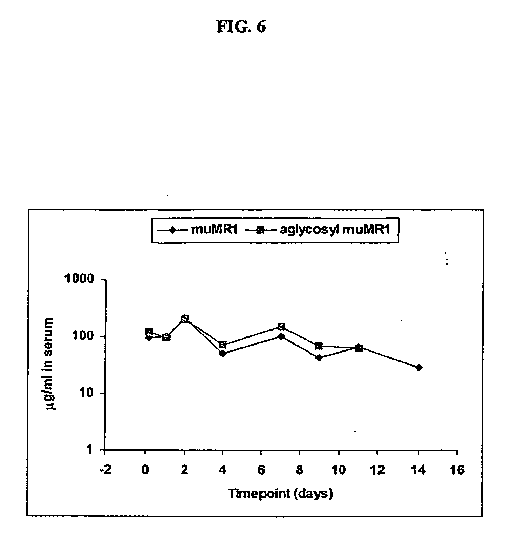

FIG. 6 illustrates the pharmacokinetics of glycosylated murine chimeric MR1 ("muMR1") and aglycosyl muMR1 antibody in BALB/c mice. Results for the glycosylated muMR1 antibody (diamond symbol) and aglycosyl muMR1 antibody (square symbol) are depicted.

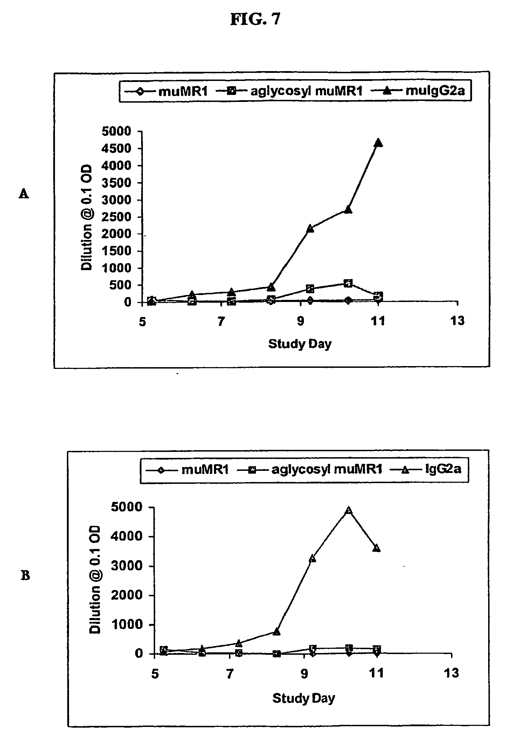

FIG. 7 (A & B) illustrates that aglycosyl anti-CD154 antibody decreases the autoantibody response to single stranded (A) and double stranded (B) DNA in SNF₁ mice. Results for the muMR1 antibody (diamond symbol) and aglycosyl muMR1 antibody (square symbol) are depicted. Control muIgG2a antibody is shown as a triangle symbol.

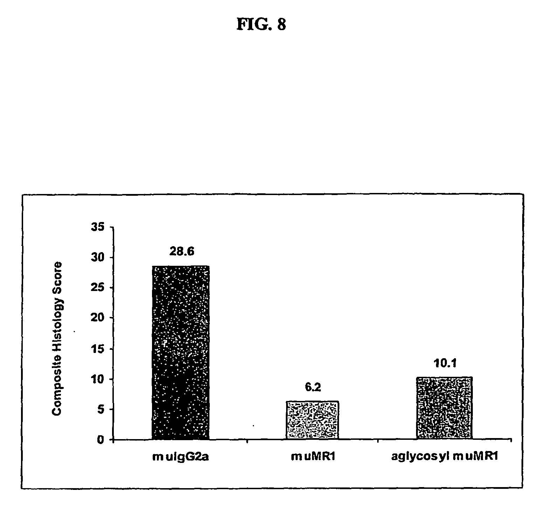

FIG. 8 illustrates that aglycosyl anti-CD154 antibody decreases the development of glomerular nephritis in SNF₁ mice. Composite histology scores are depicted for muIgG2a (controls), muMR1 and aglycosyl muMR1 treated mice.

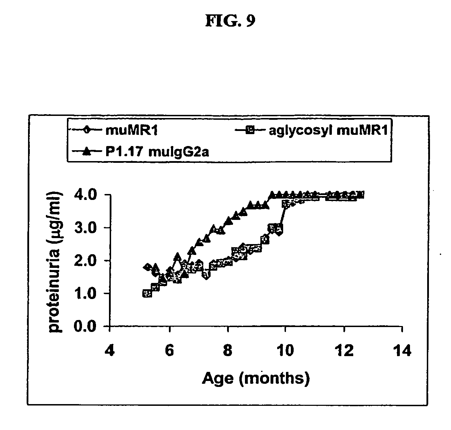

FIG. 9 illustrates that aglycosyl anti-CD154 antibodies delay the onset of glomerular nephritis in SNF1 mice. Results for the muMR1 antibody (diamond symbol) and aglycosyl muMR1 antibody (square symbol) are shown. Control muIgG2a antibody is shown as a triangle symbol.

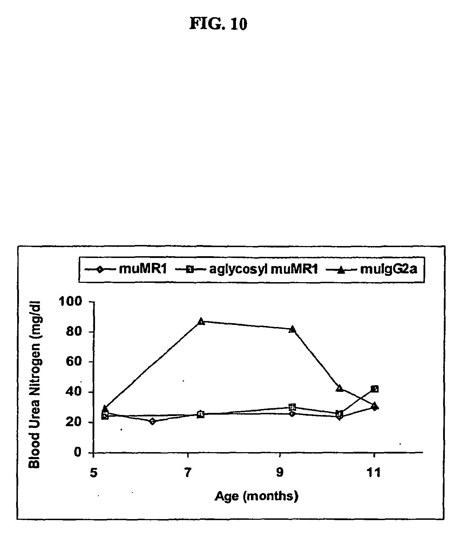

FIG. 10 illustrates that aglycosyl anti-CD154 antibodies delay onset of increased blood urea nitrogen ("BUN") levels in SNF₁ mice. Results for the muMR1 antibody (diamond symbol) and aglycosyl muMR1 antibody (square symbol) are shown. Control muIgG2a antibody is shown as a triangle symbol.

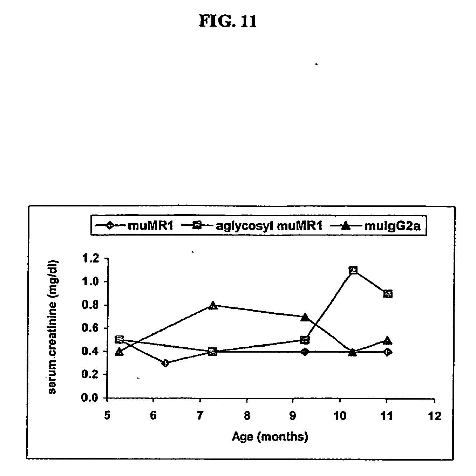

FIG. 11 illustrates that aglycosyl anti-CD154 antibodies prevent an increase of serum creatinine in SNF1 mice. Results for the muMR1 antibody (diamond symbol) and aglycosyl muMR1 antibody (square symbol) are shown. Control muIgG2a antibody is shown as a triangle symbol.

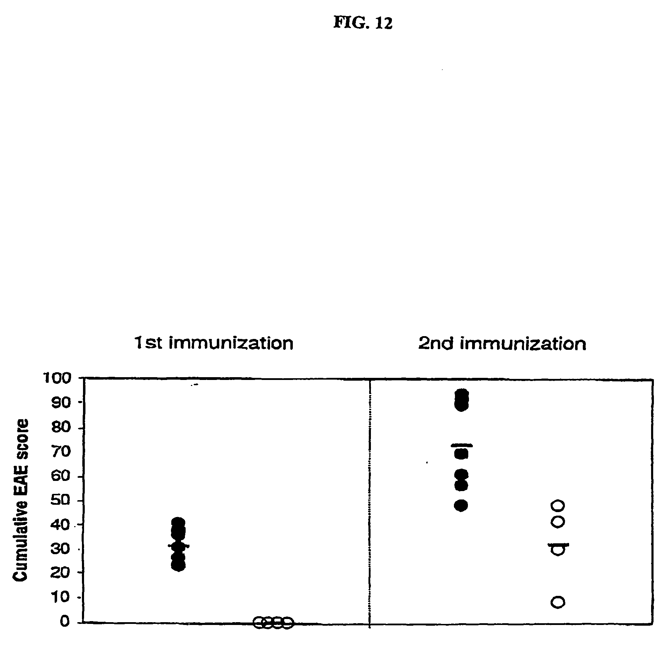

FIG. 12 illustrates that mice treated with muMR1 antibody did not develop symptoms of experimental autoimmune encephalomyelitis ("EAE"), as compared with mice treated with the isotype control P1.17 antibody. Results for the muMR1 antibody (open circles) and P1.17 control antibody (closed circles) are depicted.

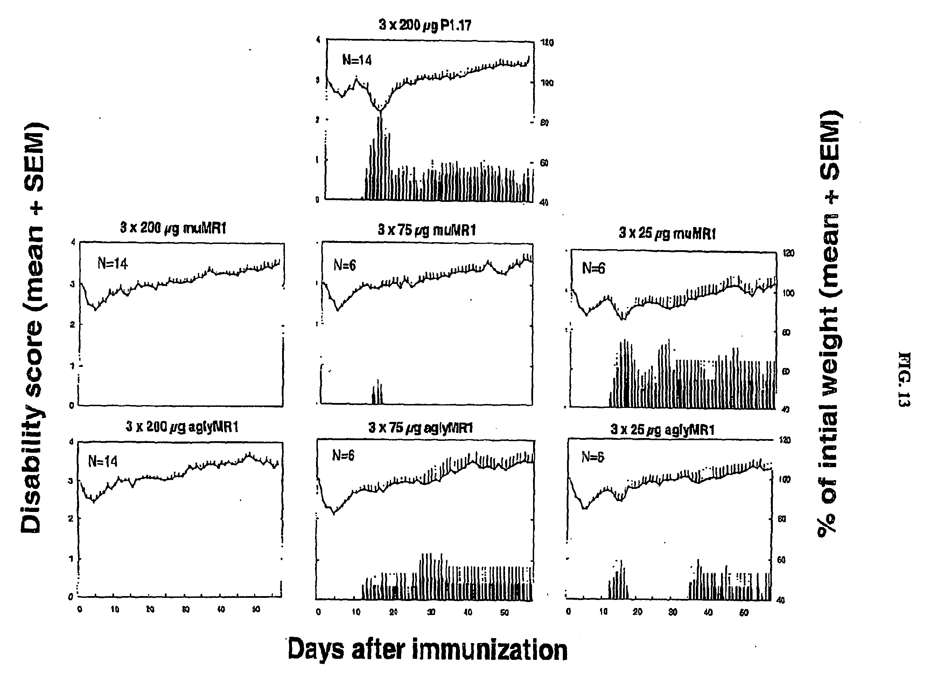

FIG. 13 illustrates that in mice treated with aglycosyl muMR1 antibody, the aglycosyl muMR1 antibody was as effective at inhibiting clinical signs of EAE as the muMR1 antibody. Results are depicted as indicating disability score (mean + standard error of mean - "SEM") and % of initial weight (mean + SEM) as related to the days following disease induction. P1.17 is a control Ig.

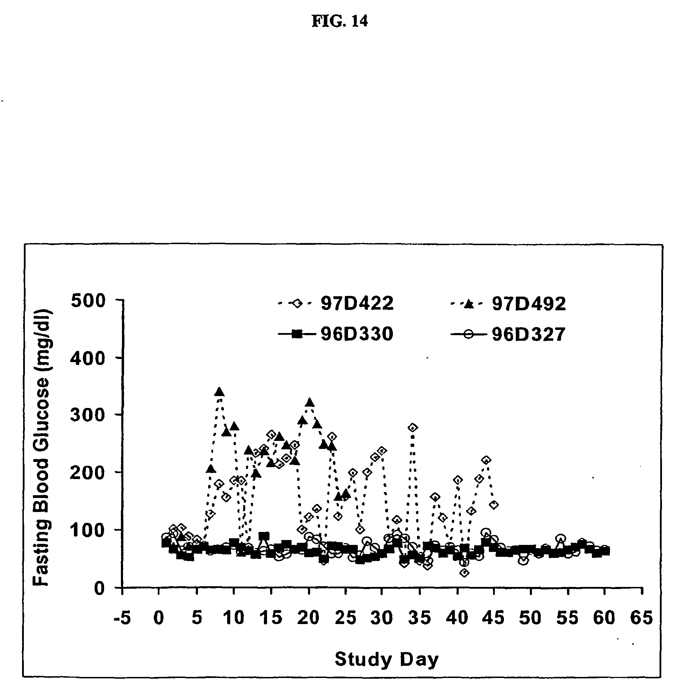

FIG. 14 depicts the fasting blood glucose ("FBG") levels in rhesus monkeys following allogeneic islet transplantation. Acute rejection was defined as FBG >100mg/dl. Aglycosyl hu5c8 mAb (dashed lines) and glycosylated hu5c8 mAb (solid lines) treated animals are depicted.

### Detailed Description Of The Invention

In order that the invention herein described may be more fully understood, the following detailed description is set forth. Unless otherwise defined, all technical and scientific terms used herein have the same meaning as commonly understood by one of ordinary skill in the art to which this invention pertains. Exemplary methods and materials are described below, although methods and materials similar or equivalent to those described herein can also be used in the practice of the present invention and will be apparent to those of skill in the art.

Throughout this application, various publications and references are referred to within brackets. Disclosures of these publications and references, in their entireties, are hereby incorporated by reference into this application to more fully describe the state of the art to which this invention pertains. The bibliographic citation for these references may be found in the text or listed by number following the Experimental Details section. In case of conflict, the present specification will control. The materials, methods, and examples are illustrative only and not intended to be limiting.

Standard reference works setting forth the general principles of recombinant DNA technology known to those of skill in the art include Ausubel et al., Current Protocols In Molecular Biology, John Wiley & Sons, New York (1998 and Supplements to 2001); Sambrook et al., Molecular Cloning: A Laboratory Manual, 2d Ed., Cold Spring Harbor Laboratory Press, Plainview, New York (1989); Kaufman et al., Eds., Handbook Of Molecular And Cellular Methods In Biology And Medicine, CRC Press, Boca Raton (1995); McPherson, Ed., Directed Mutagenesis: A Practical Approach, IRL Press, Oxford (1991).

Standard reference works setting forth the general principles of immunology known to those of skill in the art include: Harlow and Lane, Antibodies: A Laboratory Manual, 2d Ed., Cold Spring Harbor Laboratory Press, Cold Spring Harbor, N.Y. (1999); and Roitt et al., Immunology, 3d Ed., Mosby-Year Book Europe Limited, London (1993). Standard reference works setting forth the general principles of medical physiology and pharmacology known to those of skill in the art include: Fauci et al., Eds., Harrison's Principles Of Internal Medicine, 14th Ed., McGraw-Hill Companies, Inc. (1998).

The present disclosure contemplates the use of aglycosyl antibodies or antibody derivatives thereof, to inhibit immune responses and to treat diseases and conditions induced by immune responses -- for example: autoimmune disease, allergy, transplant rejection, inflammation, graft-vs-host disease, fibrosis, and atherosclerosis.

More specifically, the aglycosyl anti-CD154 antibodies used for treatment, in particular for human treatment, include human antibodies, humanized antibodies, chimeric antibodies, polyclonal antibodies and multimeric antibodies.

### ANTIBODIES

An antibody is a glycoprotein of approximate MW 150 kD, that is produced by the humoral arm of the immune system of vertebrates in response to the presence of foreign molecules in the body. A functional antibody or antibody derivative is able to recognize and bind to its specific antigen *in vitro* or *in vivo*, and may initiate any subsequent actions associated with antibody-binding, including for example, direct cytotoxicity, complement-dependent cytotoxicity ("CDC"), antibody-dependent cytotoxicity ("ADCC"), and antibody production.

Upon binding to the antigen, antibodies activate one or more of the many effector systems of the immune system that contribute to the neutralization, destruction and elimination of the infecting microorganism or other antigen-containing entity, - e.g., cancer cell.

Though naturally occurring antibodies are derived from a single species, engineered antibodies and antibody fragments may be derived from more than one species of animal, - e.g., chimeric antibodies. To date, mouse (murine)/human chimeric and murine/non-human primate antibodies have been generated, though other species' combinations are possible.

In one embodiment, the aglycosyl anti-CD154 antibodies of this invention are chimeric antibodies. Typically, chimeric antibodies include the heavy and/or light chain variable regions, including both complementary determining region ("CDR") and framework residues, of one species, (typically mouse) fused to constant regions of another species (typically human). These chimeric mouse/human antibodies contain approximately 75% human and 25% mouse amino acid sequences, respectively. The human sequences represent the constant regions of the antibody, while the mouse sequences represent the variable regions (and thus contain the antigen-binding sites) of the antibody.

The rationale for using such chimeras is to retain the antigen specificity of the mouse antibody but reduce the immunogenicity of the mouse antibody (a mouse antibody would cause an immune response against it in species other than the mouse) and thus be able to employ the chimera in human therapies.

In another specific embodiment, the aglycosyl anti-CD154 antibodies of this invention include chimeric antibodies comprising framework regions from one antibody and CDR regions from another antibody.

In a more specific embodiment, the aglycosyl anti-CD154 antibodies of this invention include chimeric antibodies comprising CDR regions from different human antibodies.

In another specific embodiment, the aglycosyl anti-CD154 antibodies of this invention include chimeric antibodies comprising CDR regions from at least two different human antibodies.

Methods of making all of the chimeric antibodies described above are well known to one of skill in the art [United States patent 5,807,715; Morrison, 1984; Sharon, 1984; Takeda, 1985].

In another embodiment of this invention, aglycosyl anti-CD154 antibodies also include primatized, humanized and fully human antibodies. Primatized and humanized antibodies typically include heavy and/or light chain CDRs from a murine antibody grafted into a non-human primate or human antibody V region framework, usually further comprising a human constant region [Riechmann, 1988; Co, 1991; United States patents 6,054,297; 5,821,337; 5,770,196; 5,766,886; 5,821,123; 5,869,619; 6,180,377; 6,013,256; 5,693,761; and 6,180,370].

### 1. Humanized Antibodies

A humanized antibody is an antibody produced by recombinant DNA technology, in which some or all of the amino acids of a human immunoglobulin light or heavy chain that are not required for antigen binding (e.g., the constant regions and the framework regions of the variable domains) are used to substitute for the corresponding amino acids from the light or heavy chain of the cognate, nonhuman antibody. By way of example, a humanized version of a murine antibody to a given antigen has on both of its heavy and light chains (1) constant regions of a human antibody; (2) framework regions from the variable domains of a human antibody; and (3) CDRs from the murine antibody. When necessary, one or more residues in the human framework regions can be changed to residues at the corresponding positions in the murine antibody so as to preserve the binding affinity of the humanized antibody to the antigen. This change is sometimes called "back mutation." Humanized antibodies generally are less likely to elicit an immune response in humans as compared to chimeric human antibodies because the former contain considerably fewer non-human components. Methods for making humanized antibodies are well know to those of skill in the art of antibodies [European Patent 239400; Jones, 1986; Riechmann, 1988; Verhoeyen, 1988; Queen, 1989; Orlandi, 1989; United States patent 6,180,370].

In one embodiment of this invention, humanized antibodies are generated by the transplantation of murine (or other non-human) CDRs onto a human antibody. More specifically, this is achieved as follows: (1) the cDNAs encoding heavy and light chain variable domains are isolated from a hybridoma; (2) the DNA sequences of the variable domains, including the CDRs, are determined by sequencing; (3) the DNAs encoding the CDRs are transferred to the corresponding regions of a human antibody heavy or light chain variable domain coding sequence by site directed mutagenesis; and (4) the human constant region gene segments of a desired isotype (e.g., 1 for CH and k for CL) are added. Finally, the humanized heavy and light chain genes are co-expressed in mammalian host cells (e.g., CHO or NS0 cells) to produce soluble humanized antibody.

At times, direct transfer of CDRs to a human framework leads to a loss of antigen-binding affinity of the resultant antibody. This is because in some cognate antibodies, certain amino acids within the framework regions interact with the CDRs and thus influence the overall antigen binding affinity of the antibody. In such cases, it would be critical to introduce "back mutations" in the framework regions of the acceptor antibody in order to retain the antigen-binding activity of the cognate antibody. The general approaches of making back mutations are well known to those of skill in the art [Queen, 1989; Co, 1991; PCT patent application WO 90/07861; Tempest, 1991].

### 2. Human Antibodies

In one embodiment of this invention, antibodies and antibody derivatives are fully human aglycosyl anti-CD154 antibodies.

In a more particular embodiment of this invention, the fully human antibodies are prepared using *in vitro*-primed human splenocytes, [Boerner, 1991] or phage-displayed antibody libraries [United States patent 6,300,064].

In a more particular embodiment of this invention, the fully human antibodies are prepared by repertoire cloning [Persson, 1991; Huang and Stollar, 1991]. In addition, United States patent 5,798,230 describes preparation of human monoclonal antibodies from human B cells, wherein human antibody-producing B cells are immortalized by infection with an Epstein-Barr virus, or a derivative thereof, that expresses Epstein-Barr virus nuclear antigen 2 ("EBNA2"), a protein required for immortalization. The EBNA2 function is subsequently shut off, resulting in an increase in antibody production.

Other methods for producing fully human antibodies involve the use of non-human animals that have inactivated endogenous Ig loci and are transgenic for un-rearranged human antibody heavy chain and light chain genes. Such transgenic animals can be immunized with activated T cells or the D1.1 protein [United States patent 5,474,771; United States patent 6,331,433; United States patent 6455,044] and hybridomas can be generated from B cells derived there from. The details of these methods are described in the art. See, e.g. the various GenPharm/Medarex (Palo Alto, CA) publications/patents concerning transgenic mice containing human Ig miniloci, including United States patent 5,789,650; the various Abgenix (Fremont, CA) publications/patents with respect to XENOMOUSE^{®} mice, including United States patents 6,075,181, 6,150,584 and 6,162,963; Green, 1997; Mendez, 1997; and the various Kirin (Japan) publications/patents concerning "transomic" mice, including European Patent 843961 and Tomizuka, 1997.

### GENERATION OF DEGLYCOSYLATED ANTIBODIES

Currently there are two ways to reduce the effector function of a mAb while retaining the other valuable attributes of the Fc portion thereof. One way to modify an antibody is to mutate amino acids on the surface of the mAb that are involved in the effector binding interactions [European Patent 239400; Jefferies, 1998]. While it is likely that some combination of mutations will lead to adequate reduction of effector function, the surface mutant antibodies that have been tested so far appear to retain residual activity. Another issue with this approach is that amino acid changes on the surface of the mAb may provoke immunogenicity.

The present invention relates to aglycosyl anti-CD154 antibodies or antibody derivatives with decreased effector function, which are characterized by a modification at the conserved N-linked site in the C_{H2} domains of the Fc portion of said antibody.

In one embodiment of present invention, the modification comprises a mutation at the heavy chain glycosylation site to prevent glycosylation at the site. Thus, in one preferred embodiment of this invention, the aglycosyl anti-CD154 antibodies or antibody derivatives are prepared by mutation of the heavy chain glycosylation site, - i.e., mutation of N298Q (N297 using Kabat EU numbering) and expressed in an appropriate host cell. For example, this mutation can be accomplished by following the manufacturer's recommended protocol for unique site mutagenesis kit from Amersham-Pharmacia Biotech (Piscataway, NJ, USA). The mutated antibody can be stably expressed in a host cell (e.g. NS0 or CHO cell) and then purified. As one example, purification can be carried out using Protein A and gel filtration chromatography. It will be apparent to those of skill in the art that additional methods of expression and purification may also be used.

In another embodiment of the present invention, the aglycosyl anti-CD154 antibodies or antibody derivatives have decreased effector function, wherein the modification at the conserved N-linked site in the C_{H2} domains of the Fc portion of said antibody or antibody derivative comprises the removal of the C_{H2} domain glycans, - i.e., deglycosylation. These aglycosyl anti-CD154 antibodies may be generated by conventional methods and then deglycosylated enzymatically. Methods for enzymatic deglycosylation of antibodies are well known to those of skill in the art [Williams, 1973; Winkelhake & Nicolson, 1976].

In another embodiment of this invention, deglycosylation may be achieved using the glycosylation inhibitor tunicamycin [Nose & Wigzell, 1983]. That is, the modification is the prevention of glycosylation at the conserved N-linked site in the C_{H2} domains of the Fc portion of said antibody.

In other embodiments of this invention, recombinant CD154 polypeptides (or cells or cell membranes containing such polypeptides) may be used as an antigen to generate an anti-CD154 antibody or antibody derivatives, which may then be deglycosylated. The antigen may be mixed with an adjuvant or linked to a hapten to increase antibody production.

Whether the modifications of the aglycosyl antibodies or antibody derivatives of the present invention are produced by the site directed mutation or by the enzymatic deglycosylation methods described above, the basics of antibody generation are well known to those skilled in the art. For example, protocols for immunizing non-human mammals are well-established in the art [Harlow, 1998; Coligan, 2001; Zola, 2000].

Following immunization, the antibodies or antibody derivatives of the present invention can be produced using any conventional technique. See, for example, Howard, 2000; Harlow, 1998; Davis, 1995; Delves, 1997; Kenney, 1997.

In some embodiments of this invention, the host cells may be, for example, (1) bacterial cells, such as E. coli, Caulobacter crescentus, Streptomyces species, and Salmonella typhimurium; (2) yeast cells, such as Saccharomyces cerevisiae, Schizosaccharomyces pombe, Pichia pastoris, Pichia methanolica; (3) insect cell lines, such as those from Spodoptera frugiperda - e.g., Sf9 and Sf21 cell lines, and expresSF^{™} cells (Protein Sciences Corp., Meriden, CT, USA) - Drosophila S2 cells, and Trichoplusia in High Five^{®} Cells (Invitrogen, Carlsbad, CA, USA); or (4) mammalian cells. Typical mammalian cells include COS1 and COS7 cells, Chinese hamster ovary (CHO) cells, NS0 myeloma cells, NIH 3T3 cells, 293 cells, HEPG2 cells, HeLa cells, L cells, HeLa, MDCK, HEK293, WI38, murine ES cell lines (e.g., from strains 129/SV, C57/BL6, DBA-1, 129/SVJ), K562, Jurkat cells, and BW5147. Other useful mammalian cell lines are well known and readily available from the American Type Culture Collection ("ATCC") (Manassas, VA, USA) and the National Institute of General Medical Sciences (NIGMS) Human Genetic Cell Repository at the Coriell Cell Repositories (Camden, NJ, USA). These cell types are only representative and this is not meant to be an exhaustive list.

In another embodiment of this invention, aglycosyl anti-CD154 antibodies or antibody derivatives are prepared by cell free translation.

In another embodiment of this invention, aglycosyl anti-CD154 antibodies or antibody derivatives are produced in bioreactors containing the antibody-expressing cells, in order to facilitate large scale production.

In another embodiment of this invention, aglycosyl anti-CD154 antibodies or antibody derivatives are produced in transgenic mammals (e.g., goats, cows, or sheep) that express the antibody in milk, in order to facilitate large scale production of aglycosyl anti-CD154 antibodies [United States patent 5,827,690; Pollock, 1999].

As noted above, the aglycosyl anti-CD154 antibodies or antibody derivatives of the present invention can be produced in prokaryotic and eukaryotic cells. The invention thus also provides cells that express the antibodies of the present invention, including hybridoma cells, B cells, plasma cells, as well as host cells recombinantly modified to express the antibodies of the present invention.

Among other considerations, some of which are described above, a host cell strain may be chosen for its ability to process the expressed CD154 protein in the desired fashion. In addition to aglycosylation, such post-translational modifications of the polypeptide include, but are not limited to, acetylation, carboxylation, phosphorylation, lipidation, and acylation, and it is an aspect of the present invention to provide aglycosyl anti-CD154 antibodies or antibody derivatives with one or more of these post-translational modifications.

### ANTIBODY MODIFICATIONS

When administered, antibodies are often cleared rapidly from the circulation and may therefore elicit relatively short-lived pharmacological activity. Consequently, frequent injections of relatively large doses of antibodies may be required to sustain the therapeutic efficacy of the antibody treatment.

In one embodiment of this invention, the aglycosyl anti-CD154 antibodies or antibody derivatives may be modified (i.e., attached to other moieties) to increase the integrity and longevity of the antibody *in vivo*. For example, the aglycosyl anti-CD154 antibodies or antibody derivatives of this invention may be modified to include a moiety that can increase stabilization, thereby prolonging the serum half-life of the antibody.

In some embodiments of this invention the aglycosyl anti-CD154 antibodies are modified by the covalent attachment of water-soluble polymers, such as polyethylene glycol, copolymers of polyethylene glycol and polypropylene glycol, carboxymethyl cellulose, dextran, polyvinyl alcohol, polyvinylpyrrolidone or polyproline -- all of which are known to exhibit substantially longer half-lives in blood following intravenous injection than do the corresponding unmodified proteins [Abuchowski, 1981; Anderson, 1992; Newmark, 1982; Katre, 1987].

Antibody modifications may also increase the protein's solubility in aqueous solution, eliminate aggregation, enhance the physical and chemical stability of the protein, and greatly reduce the immunogenicity and antigenicity of the protein. As a result, the desired *in vivo* biological activity may be achieved by the administration of such polymer-protein adducts less frequently or in lower doses than with the unmodified protein.

In some embodiments of this invention, the aglycosyl anti-CD154 antibodies are modified by labeling with a detectable marker, for example, a radioactive isotope, enzyme, dye or biotin.

In some embodiments of this invention, the aglycosyl anti-CD154 antibodies or antibody derivatives are modified by being conjugated to a therapeutic agent, for example, a radioisotope or radionuclide (e.g., 111In or 90Y), toxin moiety (e.g., tetanus toxoid or ricin), toxoid or chemotherapeutic agent [United States patent 6,307,026].

In some embodiments of this invention, the aglycosyl anti-CD154 antibodies or antibody derivatives are modified by being conjugated to an imaging agent. Imaging agents may include for example a labeling moiety (e.g., biotin, fluorescent moieties, radioactive moieties, histidine tag or other peptide tags) for easy isolation or detection.

### ANTIBODY DERIVATIVES

The present invention also relates to aglycosyl anti-CD154 antibody derivatives. All of the methods and reagents described above with respect to aglycosyl anti-CD154 antibodies may be used to produce aglycosyl anti-CD154 antibody derivatives of this invention.

In some embodiments of this invention, the aglycosyl anti-CD154 antibody derivatives include heteromeric antibody complexes and antibody fusions, such as bispecific antibodies, hemidimeric antibodies, multivalent antibodies (i.e., tetravalent antibodies) and single-chain antibodies. A hemidimeric antibody is made up of an Fc portion and one Fab portion. A single chain antibody is made up of variable regions linked by protein spacers in a single protein chain.

In some embodiments of this invention, the aglycosyl anti-CD154 antibodies derivatives of this invention may also include proteins containing one or more immunoglobulin light chains and/or heavy chains, such as monomers and homo-or hetero-multimers (e.g., dimers or trimers) of these chains, where these chains are optionally disulfide-bonded or otherwise crosslinked. These antibodies derivatives may be capable of binding to one or more antigens.

According to an alternative embodiment, the present invention includes aglycosylated antigen-binding fragments of whole antibodies, such as Fab, Fab', F(ab')2 and F(v) antibody fragments. In a further embodiment, the present invention includes antigen-binding fragments of whole antibodies, such as Fab, Fab', F(ab')2 and F(v) antibody fragments.

### CELL LINES

This invention also provides cell lines producing the aglycosyl anti-CD154 antibodies disclosed herein. One such cell line, that produces the aglycosyl hu5c8 antibody, was deposited on January 14, 2003 with the ATCC, 10801 University Blvd., Manassas, Virginia, 20110-2209, U.S.A., under the provisions of the Budapest Treaty for the International Recognition of the Deposit of Microorganism for the Purpose to Patent Procedure and assigned ATCC Accession No. PTA-4931. A second such cell line, that produces the chimeric murine, aglycosyl mu5c8 antibody, was deposited on January 14, 2003 with the ATCC, 10801 University Blvd., Manassas, Virginia, 20110-2209, U.S.A., under the provisions of the Budapest Treaty for the International Recognition of the Deposit of Microorganism for the Purpose to Patent Procedure and assigned ATCC Accession No. PTA-4934.

### THERAPEUTIC METHODS

In one embodiment of this invention, an aglycosyl anti-CD154 antibody, or an antibody derivative thereof, or a pharmaceutical composition comprising the antibody or antibody derivative, is capable of inhibiting an immune response in a subject. The antibody, antibody derivative or pharmaceutical composition is administered to the subject in an effective inhibiting amount.

An "effective inhibiting amount" of an antibody, antibody derivative or pharmaceutical composition is any amount which is effective to inhibit the CD154-CD40 interaction in the subject to whom it is administered. Methods of determining an "inhibiting amount" are well known to those skilled in the art and depend upon factors including, but not limited to: the type of subject involved, the size of the subject and the therapeutic agent delivered.

In a specific embodiment of this invention, the aglycosyl anti-CD154 antibody, antibody derivative or pharmaceutical composition comprising the antibody or antibody derivative is capable of binding to the CD154 protein molecule.

In a specific embodiment of this invention, the aglycosyl anti-CD154 antibody, antibody derivative or pharmaceutical composition comprising the antibody or antibody derivative is capable of binding to the CD154 protein that is specifically bound by aglycosyl hu5c8 produced by the cell line having ATCC Accession No. PTA-4931.

In a specific embodiment of this invention, the aglycosyl anti-CD154 antibody, antibody derivative or pharmaceutical composition comprising the antibody or antibody derivative is capable of binding to the CD154 epitope that is specifically bound by aglycosyl hu5c8 produced by the cell line having ATCC Accession No. PTA-4931, and wherein the aglycosyl anti-CD154 antibody or antibody derivative is characterized by a mutation of N298Q (N297 using EU Kabat numbering).

In a specific embodiment of this invention, the aglycosyl anti-CD154 antibody, antibody derivative or pharmaceutical composition comprising the antibody or antibody derivative does not bind to an effector receptor. In a more specific embodiment of this invention, the aglycosyl anti-CD154 antibody, antibody derivative or pharmaceutical composition comprising the antibody or antibody derivative is capable of binding to the CD154 protein that is specifically bound by aglycosyl hu5c8 produced by the cell line having ATCC Accession No. PTA-4931, and wherein the aglycosyl anti-CD154 antibody or antibody derivative or pharmaceutical composition does not bind to an effector receptor.

In a specific embodiment of this invention, the aglycosyl anti-CD154 antibody, antibody derivative or pharmaceutical composition comprising the antibody or antibody derivative does not cause thrombosis. In a more specific embodiment of this invention, the aglycosyl anti-CD154 antibody, antibody derivative or pharmaceutical composition comprising the antibody or antibody derivative is capable of binding to the CD154 protein that is specifically bound by aglycosyl hu5c8 produced by the cell line having ATCC Accession No. PTA-4931, and wherein the aglycosyl anti-CD154 antibody or antibody derivative or pharmaceutical composition does not cause thrombosis.

In another specific embodiment of this invention, the aglycosyl anti-CD154 antibody, antibody derivative or pharmaceutical composition comprising the antibody or antibody derivative is capable of inhibiting the immune response by inhibiting the CD154-CD40 interaction.

In one embodiment of this invention, the aglycosyl anti-CD154 antibody, antibody derivative or pharmaceutical composition comprising the antibody or antibody derivative is capable of inhibiting inflammation. For the purposes of this invention, inflammatory responses are characterized by redness, swelling, heat and pain, as consequences of capillary dilation with edema and migration of phagocytic leukocytes. Some examples of inflammatory responses include: arthritis, contact dermatitis, hyper-IgE syndrome, inflammatory bowel disease, allergic asthma, and idiopathic inflammatory disease [Gallin, 1989]. Some examples of arthritis include: rheumatoid arthritis, non-rheumatoid inflammatory arthritis, arthritis associated with Lyme disease and inflammatory osteoarthritis. Some examples of idiopathic inflammatory disease include: psoriasis and systemic lupus.

In one embodiment of this invention, the aglycosyl anti-CD154 antibody, antibody derivative or pharmaceutical composition comprising the antibody or antibody derivative is capable of inhibiting rejection by the subject of a transplanted organ.

In a more specific embodiment of this invention, the aglycosyl anti-CD154 antibody, antibody derivative or pharmaceutical composition comprising the antibody or antibody derivative is capable of inhibiting rejection by the subject of a transplanted heart, kidney, liver, skin, pancreatic islet cells or bone marrow.

In one embodiment of this invention, the aglycosyl anti-CD154 antibody, antibody derivative or pharmaceutical composition comprising the antibody or antibody derivative is capable of inhibiting graft-vs-host disease in a subject.

In one embodiment of this invention, the aglycosyl anti-CD154 antibody, antibody derivative or pharmaceutical composition comprising the antibody or antibody derivative is capable of inhibiting allergic responses in a subject - for example, hay fever or an allergy to penicillin or other drugs.

In one embodiment of this invention, the aglycosyl anti-CD154 antibody, antibody derivative or pharmaceutical composition comprising the antibody or antibody derivative is capable of inhibiting the autoimmune response in a subject suffering from autoimmune disease. Examples of autoimmune diseases include, but are not limited to, rheumatoid arthritis, Myasthenia gravis, systemic lupus erythematosus, Graves' disease, idiopathic thrombocytopenia purpura, hemolytic anemia, diabetes mellitus, inflammatory bowel disease, Crohn's disease, multiple sclerosis, psoriasis, and drug-induced autoimmune diseases, - e.g., drug-induced lupus.

In one embodiment of this invention, the aglycosyl antibody, antibody derivative or pharmaceutical composition comprising the antibody or antibody derivative is capable of inhibiting an autoimmune response in a subject suffering from an autoimmune response which is derived from an infectious disease.

In one embodiment of this invention, the aglycosyl antibody, antibody derivative or pharmaceutical composition comprising the antibody or antibody derivative is capable of inhibiting an autoimmune response in a subject suffering from an autoimmune response which is derived from Reiter's syndrome, spondyloarthritis, Lyme disease, HIV infection, syphilis, or tuberculosis.

In one embodiment of this invention, the aglycosyl antibody, antibody derivative or pharmaceutical composition comprising the antibody or antibody derivative is capable of inhibiting fibrosis in a subject.

Some examples of fibrosis include: pulmonary fibrosis or fibrotic disease. Some examples of pulmonary fibrosis include: pulmonary fibrosis secondary to adult respiratory distress syndrome, drug-induced pulmonary fibrosis, idiopathic pulmonary fibrosis, or hypersensitivity pneumonitis. Some examples of fibrotic diseases include: Hepatitis-C; Hepatitis-B; cirrhosis; cirrhosis of the liver secondary to a toxic insult; cirrhosis of the liver secondary to drugs; cirrhosis of the liver secondary to a viral infection; and cirrhosis of the liver secondary to an autoimmune disease.

In one embodiment of this invention, the aglycosyl antibody, antibody derivative or pharmaceutical composition comprising the antibody or antibody derivative is capable of inhibiting gastrointestinal disease. Some examples of gastrointestinal disease include: esophageal dysmotility, inflammatory bowel disease and scleroderma.

In one embodiment of this invention, the aglycosyl antibody, antibody derivative or pharmaceutical composition comprising the antibody or antibody derivative is capable of inhibiting vascular disease. Some examples of vascular disease include: atherosclerosis or reperfusion injury.

In one embodiment of this invention, the aglycosyl antibody, antibody derivative or pharmaceutical composition comprising the antibody or antibody derivative is capable of inhibiting the proliferation of T cell tumor cells in a subject suffering from a T cell cancer, - e.g., a T cell leukemia or lymphoma. Such an aglycosyl anti-CD154 antibody or antibody derivative or pharmaceutical composition may be administered to the subject in an amount effective to inhibit the proliferation of T cell tumor cells in that subject.

In one embodiment of this invention, the aglycosyl antibody, antibody derivative or pharmaceutical composition comprising the antibody or antibody derivative is capable of inhibiting viral infection of the T cells of a subject by the HTLV I virus. Such an aglycosyl anti-CD154 antibody, antibody derivative or pharmaceutical composition may be administered to the subject in an amount effective to inhibit viral infection.

In one embodiment of this invention, the aglycosyl antibody, antibody derivative or pharmaceutical composition comprising the antibody or antibody derivative is capable of imaging tumor cells or neoplastic cells in a subject that express a protein that is specifically recognized by aglycosyl hu5c8 produced by the cell line having ATCC Accession No. PTA-4931. A method for imaging tumor cells or neoplastic cells in a subject comprises the steps of: administering to the subject an effective amount of the aglycosyl anti-CD154 antibody, antibody derivative, or the pharmaceutical composition under conditions permitting the formation of a complex between the antibody or antibody derivative and a protein on the surface of tumor cells or neoplastic cells; and imaging any antibody/protein complex or antibody derivative/complex formed, thereby imaging any tumor cells or neoplastic cells in the subject.

In one embodiment of this invention, the aglycosyl antibody, antibody derivative or pharmaceutical composition comprising the antibody or antibody derivative is capable of detecting the presence of tumor cells or neoplastic cells in a subject that express a protein that is specifically recognized by aglycosyl hu5c8 produced by the cell line having ATCC Accession No. PTA-4931. One such method for detecting the presence of tumor cells or neoplastic cells in a subject comprises the steps of: administering to the subject an effective amount of aglycosyl antibody, antibody derivative, or the pharmaceutical composition under conditions permitting the formation of a complex between the antibody or antibody derivative and a protein; clearing any unbound imaging agent from the subject; and detecting the presence of any antibody/protein complex or antibody derivative/complex formed, the presence of such complex indicating the presence of tumor cells or neoplastic cells in the subject.

### PHARMACEUTICAL COMPOSITIONS

This invention provides pharmaceutical compositions comprising an aglycosyl anti-CD154 antibody or antibody derivative, as disclosed herein.

In one embodiment of this invention, the pharmaceutical composition comprises one or more aglycosyl anti-CD154 antibodies or antibody derivatives.

In another embodiment of this invention, the pharmaceutical compositions may further comprise a pharmaceutically acceptable carrier, an adjuvant, a delivery vehicle, a buffer or a stabilizer.

In a more particular embodiment of this invention, the pharmaceutically acceptable carrier is phosphate buffered saline, physiological saline, water, citrate/sucrose/Tween formulations and emulsions - e.g., oil/water emulsions.

In one instance, the pharmaceutical composition may be delivered in a microencapsulation device so as to reduce or prevent an host immune response against the protein. The antibody or antibody derivative may also be delivered microencapsulated in a membrane, such as a liposome.

In one embodiment of this invention, the pharmaceutical composition may be in the form of a sterile injectable preparation, for example, a sterile injectable aqueous or oleaginous suspension. This suspension may be formulated according to techniques known in the art using suitable dispersing, wetting, and suspending agents.

In one instance, the pharmaceutical composition may be delivered orally, topically or intravenously.

In a more specific embodiment of this invention, for oral administration, the pharmaceutical composition is formulated in a suitable capsule, tablet, aqueous suspension or solution. Solid formulations of the compositions for oral administration can contain suitable carriers or excipients, such as corn starch, gelatin, lactose, acacia, sucrose, microcrystalline cellulose, kaolin, mannitol, dicalcium phosphate, calcium carbonate, sodium chloride, or alginic acid. Disintegrators that can be used include, without limitation, microcrystalline cellulose, corn starch, sodium starch glycolate, and alginic acid. Tablet binders that can be used include acacia, methylcellulose, sodium carboxymethylcellulose, polyvinylpyrrolidone(Povidone^{™}), hydroxypropyl methylcellulose, sucrose, starch, and ethylcellulose. Lubricants that can be used include magnesium stearates, stearic acid, silicone fluid, talc, waxes, oils, and colloidal silica.

In a more specific embodiment of this invention, for topical applications, the pharmaceutical compositions may be formulated in a suitable ointment. Some examples of formulations of a composition for topical use include: drops, tinctures, lotions, creams, solutions, and ointments containing the active ingredient and various supports and vehicles.

In one embodiment of this invention, a topical semi-solid ointment formulation typically comprises a concentration of the active ingredient from about 1 to 20%, - e.g., 5 to 10%, in a carrier, such as a pharmaceutical cream base.

In one embodiment of this invention, pharmaceutical compositions for inhalation and transdermal compositions can also readily be prepared.

In one embodiment of this invention, liquid formulations of a pharmaceutical composition for oral administration prepared in water or other aqueous vehicles can contain various suspending agents such as methylcellulose, alginates, tragacanth, pectin, kelgin, carrageenan, acacia, polyvinylpyrrolidone, and polyvinyl alcohol. Liquid formulations of pharmaceutical compositions of this invention can also include solutions, emulsions, syrups and elixirs containing, together with the active compound(s), wetting agents, sweeteners, and coloring and flavoring agents. Various liquid and powder formulations of the pharmaceutical compositions can be prepared by conventional methods for inhalation into the lungs of the mammal to be treated.

In one embodiment of this invention, liquid formulations of a pharmaceutical composition for injection can comprise various carriers such as vegetable oils, dimethylacetamide, dimethylformamide, ethyl lactate, ethyl carbonate, isopropyl myristate, ethanol, polyols - i.e., glycerol, propylene glycol, liquid polyethylene glycol, and the like. In some embodiments, the composition includes a citrate/sucrose/tween carrier. For intravenous injections, water soluble versions of the compositions can be administered by the drip method, whereby a pharmaceutical formulation containing the antifungal agent and a physiologically acceptable excipient is infused. Physiologically acceptable excipients can include, for example, 5% dextrose, 0.9% saline, Ringer's solution or other suitable excipients. A suitable insoluble form of the composition can be prepared and administered as a suspension in an aqueous base or a pharmaceutically acceptable oil base, such as an ester of a long chain fatty acid - e.g., ethyl oleate.

In one embodiment of this invention, the pharmaceutical composition comprises from about 0.1 to 90% by weight (such as 1 to 20% or 1 to 10%) of an aglycosyl anti-CD154 antibody or antibody derivative thereof, in a pharmaceutically acceptable carrier.

In one embodiment of this invention, the optimal percentage of the antibody or antibody derivative in each pharmaceutical composition varies according to the formulation itself and the therapeutic effect desired in the specific pathologies and correlated therapeutic regimens. Pharmaceutical formulation is well-established in the art [Gennaro, 2000; Ansel, 1999; Kibbe, 2000]. Conventional methods, known to those of ordinary skill in the art of medicine, can be used to administer the pharmaceutical composition to the subject.

In some embodiments of this invention, the pharmaceutical composition further comprises an immunosuppressive or immunomodulatory compound. For example, such an immunosuppressive or immunomodulatory compound may be one of the following: an agent that interrupts T cell costimulatory signaling via CD28; an agent that interrupts calcineurin signaling, a corticosteroid, an anti-proliferative agent, and an antibody that specifically binds to a protein expressed on the surface of immune cells, including but not limited to CD45, CD2, IL2R, CD4, CD8 and RANK FcR, B7, CTLA4, TNF, LTβ, and VLA-4.

In some embodiments of this invention, the immunosuppressive or immunomodulatory compound is tacrolimus, sirolimus, mycophenolate mofetil, mizorubine, deoxyspergualin, brequinar sodium, leflunomide, rapamycin or azaspirane.

In other embodiments of this invention, antibodies, antibody derivatives or pharmaceutical compositions comprising them may be included in a container, package or dispenser alone or as part of a kit with labels and instructions for administration.

### ADMINISTRATION AND DELIVERY ROUTES

The aglycosyl anti-CD154 antibodies or antibody derivatives thereof, and pharmaceutical compositions of this invention may be administered to a subject in any manner which is medically acceptable. For the purposes of this invention, "administration" means any of the standard methods of administering an antibody, antibody derivative or pharmaceutical composition known to those skilled in the art, and should not be limited to the example provide herein.

In some instances, the aglycosyl anti-CD154 antibodies, antibody derivatives and pharmaceutical compositions may be administered to a subject by injection intravenously, subcutaneously, intraperitoneally, intramuscularly, intramedullarily, intraventricularly, intraepidurally, intraarterially, intravascularly, intra-articularly, intra-synovially, intrasternally, intrathecally, intrahepatically, intraspinally, intratumorly, intracranially, enteral, intrapulmonary, transmucosal, intrauterine, sublingual, or locally at sites of inflammation or tumor growth by using standard methods.

In some instances, the aglycosyl anti-CD154 antibodies, antibody derivatives and pharmaceutical compositions may be administered to a subject by routes including oral, nasal, ophthalmic, rectal, or topical.

In a more specific instance, the aglycosyl anti-CD154 antibodies, antibody derivatives and pharmaceutical compositions of this invention may be administered to a subject orally in the form of capsules, tablets, aqueous suspensions or solutions.

In a more specific instance, the aglycosyl anti-CD154 antibodies, antibody derivatives and pharmaceutical compositions may be administered to a subject topically by application of a cream, ointment or the like.

In other instances, the aglycosyl anti-CD154 antibodies, antibody derivatives and pharmaceutical compositions of this invention may also be administered by inhalation through the use of a nebulizer, a dry powder inhaler or a metered dose inhaler.

In further instances, the aglycosyl anti-CD154 antibodies, antibody derivatives and pharmaceutical compositions may be administered to a subject by sustained release administration, by such means as depot injections of erodible implants directly applied during surgery or by implantation of an infusion pump or a biocompatible sustained release implant into the subject.

In a more specific instance, the aglycosyl anti-CD154 antibodies, antibody derivatives and pharmaceutical compositions of this invention may be administered to a subject by injectable depot routes of administration, such as by using 1-, 3-, or 6-month depot injectable or biodegradable materials and methods.

In a more specific instance, the aglycosyl anti-CD154 antibodies, antibody derivatives and pharmaceutical compositions of this invention may be administered to a subject by applying to the skin of the subject a transdermal patch containing the antibody, antibody derivative or pharmaceutical composition, and leaving the patch in contact with the subject's skin, generally for 1 to 5 hours per patch.

In other instances, the aglycosyl anti-CD154 antibodies, antibody derivatives and pharmaceutical compositions may be administered to a subject at any dose per body weight and any dosage frequency that is medically acceptable. Acceptable dosage includes a range of between about 0.01 and 200 mg/kg subject body weight.

In further instances, the aglycosyl anti-CD154 antibodies, antibody derivatives and pharmaceutical compositions of this invention may be administered to a subject repeatedly at intervals ranging from each day to every other month.

In one instance, the aglycosyl anti-CD154 antibodies, antibody derivatives and pharmaceutical compositions can be administered in multiple doses per day, if desired, to achieve the total desired daily dose. The effectiveness of the method of treatment can be assessed by monitoring the subject for known signs or symptoms of a disorder.

For all instances, the dosage and dose rate of the aglycosyl anti-CD154 antibodies, antibody derivatives thereof and pharmaceutical compositions of this invention effective to produce the desired effects will depend on a variety of factors, such as the nature of the disease to be treated, the size of the subject, the goal of the treatment, the specific pharmaceutical composition used, and the judgment of the treating physician.

The aglycosyl anti-CD154 antibodies, antibody derivatives thereof and pharmaceutical compositions of this invention may be administered as a single dosage for certain indications, such as preventing immune response to an antigen to which a subject is exposed for a brief time, such as an exogenous antigen administered on a single day of treatment. Examples of such a therapy would include coadministration of the antibody or antibody derivative of the invention along with a therapeutic agent, for example an antigenic pharmaceutical, an allergen or a blood product, or a gene therapy vector. In indications where antigen is chronically present, such as in controlling immune reaction to transplanted tissue or to chronically administered antigenic pharmaceuticals, the antibodies, antibody derivatives or pharmaceutical compositions of the invention are administered at intervals for as long a time as medically indicated, ranging from days or weeks to the life of the subject.

In one instance, the subject(s) that can be treated by the above-described methods is an animal. Preferably, the animal is a mammal. Examples of mammals that may be treated include, but are not limited to, humans, non-human primates, rodents (including rats, mice, hamsters and guinea pigs) cow, horse, sheep, goat, pig, dog and cat. Preferably, the mammal is a human.

This invention may be better understood based on the Examples that follow. However, one skilled in the art will readily appreciate that the specific methods and results discussed are merely illustrative of the invention as described more fully in the Embodiments of the Invention that follow thereafter.

### Experimental Details

### EXAMPLES

The following examples illustrate the products of the present invention and their uses. Suitable modifications and adaptations of the described conditions and parameters normally encountered in the art of molecular biology that are apparent to those skilled in the art are within the spirit and scope of the present invention.

### Example 1: Generation and Evaluation of Aglycosyl hu5c8 Antibody

### Expression and characterization of aglycosyl hu5c8 mAb

In order to reduce the effector function of hu5c8 mAb, an aglycosyl form was created by changing the canonical N-linked Asn site in the heavy chain C_{H2} domain to a Gln residue.

A competitive binding assay demonstrated that the ability of the aglycosyl hu5c8 mAb to bind to cell-surface CD154 was unaltered, as compared with the glycosylated hu5c8 mAb (FIG. 1).

The reduction in effector function was measured *in vitro* using a bridging assay format. The relative binding of aglycosyl hu5c8 mAb to FcγRI was diminished twenty-five-fold, as compared with glycosylated hu5c8 mAb (FIG. 2A). No residual binding of the aglycosyl hu5c8 mAb to FcγRIII could be demonstrated at concentrations up to 5 mg/ml, while the normal glycosylated hu5c8 mAb gave an EC50 of 50 ng/ml in the same assay format (FIG. 2B).

### Pharmacokinetics of aglycosyl hu5c8 mAb in cynomolgus monkeys

The serum concentration-time profiles after a single 20 mg/kg dose of hu5c8 mAb and aglycosyl hu5c8 mAb from two independent studies were subjected to pharmacokinetic analysis using a two compartment model with a first order elimination rate constant (WinNolin Professional Software v3.1, Pharsight Corp., Cary, NC). FIG. 3 contains the pharmacokinetic profiles and Table 1 contains the mean pharmacokinetic parameters for hu5c8 mAb and aglycosyl hu5c8 mAb. The clearance and volume of distribution for hu5c8 mAb was slightly greater than for aglycosyl hu5c8 mAb.

**Table 1**

| Mean Pharmacokinetic Parameters Following Single 20mg/kg Intravenous Doses of Either hu5c8 or Aglycosyl hu5c8 to Cynomolgus Monkeys^{A} | | | | |
|---|---|---|---|---|
| Antibody | Cmax^{B} (µg/mL) | Cl^{C} (mLhr/kg) | Vss^{D} (mL/kg) | t_{1/2}^{E} (d) |
| hu5c8 | 515(±16) | 4.61(±0.70) | 71 (±10) | 11.5(±2.5) |
| | | | | |
| agly. hu5c8 | 869(±360) | 3.10(±1.10) | 47(±11) | 11.8(±3.0) |

| | | | | |
|---|---|---|---|---|
| ^{A}Data reported as the arithmetic mean ± standard deviation, n = 3 for the hu5c8 treatment group, n = 4 for the aglycosyl hu5c8 treatment group. ^{B}maximum serum concentration, systemic clearance, ^{D}volume of distrubution at steady state, ^{E} terminal phase serum half-life. | | | | |

### METHODS - Example 1

1. Generation of Antibodies. The selection, cloning, and humanization of hu5c8 mAb have been described previously. See Lederman, 1992 and Karpusas, 2001, respectively. The hu5c8 mAb hyridoma is available from the ATCC (HB10916). The heavy chain glycosylation site mutation N298Q (N297 using EU numbering) was made in glycosylated hu5c8 mAb by unique site elimination mutagenesis using a kit from Amersham-Pharmacia Biotech (Piscataway, NJ, USA) following the manufacturer's recommended protocol. The resulting aglycosyl hu5c8 was stably expressed in NS0 myeloma cells and purified by Protein A and gel filtration chromatography. The cell line producing the aglycosyl hu5c8 antibody is available from the ATCC (PTA-4931). SDS-PAGE and analytical gel filtration chromatography demonstrated that the protein formed the expected disulfide linked tetramer.

2. CD154 binding assay. A FACS-based competitive binding assay was done on huCD154⁺ D1.1 cells (gift of Dr. Leonard Chess, Columbia University, also available from the ATCC (CRL-10915)). The binding of 0.1 mg/ml of biotinylated hu5c8 mAb to cell surface CD154 was competed with titrations of hu5c8 mAb and aglycosyl hu5c8 mAb. Cell-bound biotinylated hu5c8 mAb was detected with streptavidin-phycoerytherin (PE) (BD-PharMingen San Diego, CA, USA). Relative binding affinities were inferred from the IC₅₀ values of four parameter curve fits.

3. CD154-FcγR bridging assays. FcγR binding affinities were measured using assays based on the ability of the antibody to form a "bridge" between antigen and a FcγR bearing cell (see below). The FcγRI (CD64) bridging assay was performed by coating 96-well Maxisorb ELISA plates (Nalge-Nunc Rochester, NY, USA) overnight at 4°C with 1 mg/ml recombinant soluble human CD154 (Biogen, Karpusas, 1995) in PBS and then blocking with 1% BSA in PBS. Titrations of hu5c8 mAb (glycosylated or aglycosyl) were then bound to CD154 for 30 minutes at 37°C, the plates were washed, and the binding of fluorescently labeled U937 (CD64⁺) cells was measured. The U937 cells were grown in RPMI medium with 10% FBS, 10 mM HEPES, L-glutamine, and penicillin/streptomycin, split 1:2, and activated for one day prior to the assay with 1000 units/ml of IFNγ to increase FcγRI expression.

The FcγRIII (CD16) bridging assays were performed using a monolayer of CD154-expressing Chinese Hamster Ovary (CHO) cells (Biogen) grown in 96-well tissue culture plates (Corning Life Sciences Acton, MA, USA), with measurement of the mAb-dependent binding of fluorescently labeled Jurkat cells transfected with CD16 (gift of Dana Farber Institute, Boston, MA, USA). The CHO-CD154⁺ cells, were seeded into 96-well plates at 1x10⁵ cells/ml and grown to confluency in α-MEM with 10% dialyzed FBS, 100 nM methotrexate, L-glutamine, and penicillin/streptomycin (all reagents from Gibco-BRL Rockville, MD, USA). CD16⁺ Jurkat cells, growing in RPMI with 10% FBS, 400 mg/ml Geneticin, 10mM HEPES, sodium pyruvate, L-glutamine, and penicillin/streptomycin (all reagents from Gibco-BRL), were split 1:2 one day prior to performing the assay.

In the assays for both the FcγRI and FcγRIII receptors, the Fc receptor-bearing cells were labeled with 2',7'-bis-(2-carboxyethyl)-5-(and-6)-carboxyfluorescein acetoxymethyl ester (BCECF-AM) (Molecular Probes Eugene, OR, USA) for 20 minutes at 37°C. After washing to remove excess BCECF-AM, 1x10⁵ of the labeled cells were incubated in the assay for 30 minutes at 37°C. Unbound FcγR⁺ cells were removed by washing several times and plates were read on a Cytofluor 2350 Fluorescent Microplate Reader (Millipore Corporation Bedford, MA, USA) with an excitation wavelength of 485 nm and an emission wavelength of 530 nm.

### Example 2: Aglycosyl hu5c8 Antibody Inhibits Primary and Secondary Humoral Responses

### Inhibition of a primary humoral response to tetanus toxoid (TT) antigen in cynomolgus monkeys

The ability of a single 20 mg/kg dose of each of aglycosyl hu5c8 mAb and glycosylated hu5c8 mAb, prepared according to Example 1, to inhibit a primary antibody response to TT was evaluated in separate studies. The administration of aglycosyl hu5c8 mAb or glycosylated hu5c8 mAb produced 70% and 77% reductions, respectively, in the overall primary immune response (E_{AUC}), as compared to saline-treated control groups. FIG. 4 shows the TT antibody titers through day 42 in graphic form, demonstrating that aglycosyl hu5c8 mAb inhibits a primary humoral response to a degree comparable to glycosylated hu5c8 mAb, despite its decreased FcγR binding capabilities.

The immunogenicity of humanized mAbs is another measure of their efficacy in this non-human primate model. Three of the four animals treated with a single 20 mg/kg dose of aglycosyl hu5c8 mAb developed a low titer of anti-hu5c8 antibodies around day 82, shortly after the drug was cleared from the serum, consistent with inhibition of the humoral response while the aglycosyl mAb was present (data not shown).

As a further measure of primary response inhibition, inguinal lymph node biopsies showed that the presence of germinal centers ("GC") in the aglycosyl hu5c8 mAb treated animals was greatly decreased, as compared to controls. In the treated animals, the GC were rare and small, occupying less than 20% of the cortex. Control animals had multiple GC with moderate to markedly reactive secondary follicles. The moderate to severe degree of lymph node hypoplasia observed was consistent with that previously observed with glycosylated hu5c8 mAb (data not shown).

No gross changes were observed in hematological parameters after the administration of a single 20 mg/kg dose of glycosylated or aglycosyl hu5c8 mAb to cynomolgus monkeys and there was no significant change in the total number of lymphocytes. Furthermore, the CD4/CD8 T cell ratio remained constant, indicating that widespread CD4⁺ T cell depletion did not occur (data not shown).

***Inhibition of a secondary humoral response to tetanus toxoid (TT) antigen in cynomolgus monkeys***

The ability of a single 20 mg/kg dose of aglycosyl hu5c8 mAb to inhibit a secondary immune response was evaluated by giving a second TT challenge to the eight saline control animals who had a normal primary response in the previously described phase of the aglycosyl hu5c8 mAb study. Prior to the second TT challenge, four of these animals received 20 mg/kg of aglycosyl hu5c8 mAb (Group 1B) and four received saline (Group 1A).

Secondary immune responses are characterized by quicker onset and are of a higher magnitude than primary immune responses. The magnitude of the secondary response relative to the primary response for individual animals (E_{AUC} secondary/ E_{AUC} primary) was calculated (Table 2). FIG. 5 shows the overall primary and secondary individual immune responses. It should be noted that there was considerable variability in the degree of immune response among the individual animals. On average, a secondary TT challenge in the saline controls (Group 1A) produced an overall antibody response that was 6.5 times higher than their primary response to this antigen, whereas animals receiving aglycosyl hu5c8 mAb (Group 1B) produced an overall secondary antibody response that was, on average, only 2.0 times higher than their primary response. Therefore, administration of aglycosyl hu5c8 mAb resulted in a 70% reduction in the magnitude of the secondary antibody response, as compared to saline controls.

**Table 2**

| Overall primary and secondary immune responses (E_{AUC}) to Tetanus Toxoid in cynomolgus monkeys. | | | | | | | | |
|---|---|---|---|---|---|---|---|---|
| Treatment Group | Group 1A (saline - saline)^{A} | | | | Group 1B (saline - aglycosyl hu5c8)^{B} | | | |
| Animal # | 1A-1 | 1A-2 | 1A-3 | 1A-4 | 1B-1 | 1B-2 | 1B-3 | 1B-4 |
| Primary E_{AUC} (x 10⁵) | 2.1 | 1.1 | 2.0 | 0.3 | 3.9 | 0.9 | 1.6 | 2.6 |
| Secondary E_{AUC} (x 10⁵) | 8.7 | 5.2 | 9.6 | 6.0 | 8.3 | 1.5 | 3.3 | 5.5 |
| Magnitude | 4.2 | 4.8 | 4.8 | 18.8 | 2.1 | 1.6 | 2.1 | 2.2 |
| Group Average Magnitude | 6.5 | | | | 2.0 | | | |

| | | | | | | | | |
|---|---|---|---|---|---|---|---|---|
| ^{A} Group 1A animals received saline prior to both the primary and the secondary TT challenge. Group 1B animals received saline prior to the primary TT challenge and aglycosyl hu5c8 prior to the secondary TT challenge.^{B} The magnitude of the secondary response is represented by the ratio of secondary E_{AUC} /primary E_{AUC}. | | | | | | | | |

### METHODS - Example 2

1. Humoral immune response to TT. Two independent studies were carried out in cynomolgus monkeys, using the same monkeys as in Example 1. Serum samples from each study were collected and whole blood for immunophenotyping was maintained at ambient temperature and analysis was performed on the day it was drawn.

This aglycosyl hu5c8 mAb study included two treatment groups. Group 1, consisting of four males and four females, received saline on day 1 and served as untreated controls. Group 2, consisting of two males and two females, received a single 20 mg/kg dose of aglycosyl hu5c8 mAb intravenously on day 1 (described above). Four hours after treatment, all animals received an intramuscular (IM) dose of 5 Lf (limes flocculating dose) of adsorbed TT. Blood was collected both pre- and post-treatment on day 1 and on selected days up to 190 days post-dosing. Lymph node biopsy samples were taken on day 15.

The glycosylated hu5c8 mAb study was comprised of five treatment groups, each containing three females. On day 1, Group 1 received saline (untreated controls) and Groups 2 through 5 received a single dose of 0.2, 1, 5 or 20 mg/kg of glycosylated hu5c8 mAb respectively, available from the ATCC (CRL-10915). Four hours after treatment, all animals received a single IM injection of 5 Lf of adsorbed TT. Blood was collected from all groups both pre- and post-treatment on day 1 and on selected days up to 42 days post dosing. To allow comparability of these two independent studies, selected serum samples were analyzed side-by-side in the anti-TT ELISA.

On day 230 after the primary TT challenge in the aglycosyl study described above, the control Group 1 was divided into two groups. Group 1A served as untreated controls, while Group 1B was treated with aglycosyl hu5c8 mAb to evaluate its ability to inhibit a secondary immune response. Animals were treated as follows: Group 1B received a single 20 mg/kg dose of aglycosyl hu5c8 mAb intravenously on day 1. Group 1A received an intravenous dose volume equivalent of phosphate buffered saline on day 1. Four hours after treatment, all animals received an IM dose of 5 Lf of adsorbed TT. Blood was collected both pre- and post-treatment on day 1 and on selected days up to 85 days post-dosing.

2. Evaluation of Immune Responses. Immune responses were evaluated using noncompartmental analysis. The immune parameters calculated included the maximum titer value (Emax), the time to reach this maximum value (tmax), and the overall antibody response to the administered antigen from time of antigen administration to the last sampling time point (EAUC(0-last)). Pharmacokinetic analysis was performed using a two compartment model with a first order elimination rate constant (WinNolin Professional Software v3.1, Pharsight Corp., Cary, NC, USA). The pharmacokinetic parameters determined include the maximum serum concentration (Cmax), the rate of systemic clearance (C1), the volume of distribution at steady state (Vss) and the terminal phase half-life (t1/2) of the antibody. Statistical analyses, including arithmetic mean, standard deviation and geometric mean were performed using Microsoft Excel version 5.0 software (Microsoft Corp., Redmond, WA, USA).

3. Immunophenotyping of Cynomolgus Monkeys. Lymphocyte immunophenotyping was performed using a two-color, whole blood staining protocol followed by FACS analysis. Briefly, 100 µl of EDTA-treated whole blood was incubated for 20 min at room temperature with one of the following combinations of labeled mAbs: CD20-FITC clone 2H7 (BD-Pharmingen San Diego CA, USA) and CD2-PE clone RPA-2.10 (BD-Pharmingen), CD3-FITC clone SP-34 (BD-Pharmingen) and CD4-PE clone OKT4 (Ortho Diagnostic Systems Raritan, NJ, USA), or CD3-FITC and CD8-PE clone DK-25 (Dako Corporation Carpinteria, CA, USA). Erythrocytes were lysed with 2 ml of 1X FACS lysing solution (Becton-Dickinson Franklin Lakes, NJ, USA). Lymphocytes were fixed with 1% paraformaldehyde and analyzed with a FACScan equipped with Cellquest software (Becton-Dickinson). The number of total lymphocytes was determined by summing all of the CD2 and CD20 positive cells. B cells were identified as CD20 positive cells. T cell subsets were identified as double positives for CD3 and CD4 or CD3 and CD8. Total lymphocytes, B cells, CD4⁺ T cells, and CD8⁺ T cells were represented as the percent of positive cells within the lymphocyte analysis gate. The CD4/CD8 ratio was calculated for each data set.

4. ELISA for hu5c8 mAb Pharmacokinetics. ELISA plates (Nalge-Nunc Rochester, NY, USA) were coated with 5 µg/ml of recombinant soluble human CD154 (Biogen, see also Karpusas 1995) in PBS overnight at 4°C and blocked with 2% donkey serum. (Jackson ImmunoResearch Laboratories West Grove, PA, USA - Catalog #017-000-121). Serial dilutions of serum and a standard curve of hu5c8 mAb from 8-500 ng/ml were captured during a one-hour incubation at room temperature. Bound hu5c8 mAb was detected using donkey anti-human IgG horseradish peroxidase (HRP) (Jackson ImmunoResearch Laboratories West Grove, PA, USA) followed by development with a 3,3',5,5'-tetramethylbenzidine ("TMB") Substrate Kit (Pierce Biotechnology Rockland, IL, USA). Plates were read at 450 nm using a Spectromax plate reader (Molecular Devices Sunnyvale, CA, USA). Softmax Pro Software (Molecular Devices) was used to back-fit the diluted serum to the linear portion of a four-parameter curve fit of the standards.

5. ELISA to Determine anti-hu5c8 Antibody Responses. ELISA plates (Corning-Costar) were coated with 1 µg/ml of aglycosyl hu5c8 mAb (described above) in bicarbonate buffer pH 9.6 overnight at 4°C and blocked with 1% BSA. Serial dilutions of cynomolgus monkey serum were incubated for 1.5 h at room temperature. The bound anti-hu5c8 mAb was detected using 100 ng/ml of biotinylated hu5c8 mAb followed by streptavidin-HRP (Pierce Biotechnology) and development with a TMB Substrate Kit (Pierce Biotechnology). Plates were read at 450 nm using a Spectromax plate reader (Molecular Devices). Antibody titer was defined as the reciprocal of the highest dilution that yielded >0.100 O.D. units over prebleed values.

6. ELISA for the Anti-TT Response. ELISA plates (Corning-Costar) were coated with 5 µg/ml of TT (Massachusetts Public Health Biologic Laboratories Boston, MA, USA) in bicarbonate buffer pH 9.6 overnight at 4°C. Serially diluted cynomolgus serum was added to the blocked plate for 2 hours at room temperature. Bound anti-TT antibodies were detected using a rabbit anti-monkey IgG-HRP (Cappel-Organon Teknika Durham, NC, USA) followed by development with a TMB Substrate Kit (Pierce Biotechnology). Plates were read at 450 nm using a Spectromax plate reader (Molecular Devices). Softmax Pro software (Molecular Devices) was used to create a four-parameter curve fit for each serially diluted serum sample. Antibody titer was defined as the reciprocal of the dilution that yielded 0.100 OD units over prebleed values.

7. Hematology. Potassium EDTA anticoagulated blood samples were collected and analyzed monthly for the following hematological parameters: total leukocyte count, erythrocyte count, hemoglobin concentration, hematocrit value, mean corpuscular volume, mean corpuscular hemoglobin, mean corpuscular hemoglobin concentration, platelet count and blood smear evaluation (including differentials).

8. Lymph node biopsy. An inguinal lymph node was collected from all animals on day 15 of the aglycosyl hu5c8 mAb, primary TT-response study. Lymph node tissue was trimmed, embedded in paraffin, sectioned and mounted onto glass slides. Slides were stained with hematoxylin and eosin. The slides were visually analyzed for the presence of the germinal centers and quantitatively assessed based on the frequency and size of germinal centers.

### Example 3: Aglycosyl muMR1 Antibody Inhibits Lupus Nephritis

Systemic lupus erythematosus ("SLE") is a spontaneously arising autoimmune disease, with a female predominance, that is characterized by the production of a variety of pathogenic anti-nuclear autoantibodies. In lupus nephritis, kidney damage is mediated by both cellular and humoral immune mechanisms, including the formation of immune complexes that deposit in kidney glomeruli and activate the complement cascade, resulting in glomerulonephritis. It has previously been established that the production of anti-nuclear autoantibodies in both human and mouse SLE is driven by cognate interactions between select populations of autoimmune Th cells and B cells. [Kalled *et al*., 1998].

Prior studies have demonstrated that for (SWR x NZB) F1 (SNF1) mice with established lupus nephritis, long-term treatment with the anti-CD154 mAb, hamster MR1 (haMR1) , beginning at 5.5 months of age prolonged survival and decreased the incidence of severe nephritis.

In this example, the efficacy of two murine chimeric MR1 mAbs in this model is described. Murine chimeric MR1 ("muMRl") consists of the original hamster heavy and light chain variable domains fused to murine IgG2a heavy and kappa light chain constant domains. An aglycosyl version of the muMRl was created by mutation of the N-linked glycosylation site in the C_{H2} domain of the IgG2a Fc. Using these two antibodies, the role of Fc glycosylation on the potency of anti-CD154 mAbs was evaluated in lupus nephritis.

The results of this example demonstrate that both of the chimeric mAbs, muMR1 and aglycosyl muMR1, retained the ability to inhibit autoantibody responses. Specifically, similar to the wild type, parent hamster MR1, both murinized antibodies retained the ability to diminish kidney inflammation, fibrosis, sclerosis, and vasculitis in mice treated with them, as compared to mice treated with a murine IgG2a control mAb.

### Pharmacokinetics of muMR1 and aglycosyl muMR1

An analysis of the pharmacokinetics of the muMR1 and aglycosyl muMR1 antibodies revealed that both antibodies exhibited the same kinetic profile in serum of BALB/c mice (FIG. 6). Specifically, the half-life of these chimeric molecules in normal mice is estimated to be ~9 days, similar to hamster MR1 (data not shown).

### Analysts of Autoantibody Responses

Treatment with muMR1 or aglycosyl muMR1 greatly diminished the autoantibody response to both dsDNA and ssDNA as compared to control animals (FIG. 7 A & B).

### Histological Analysis

An analysis of the histology of the kidney demonstrated that three of the five isotype control animals had severe end-stage nephropathy characterized by the numerous features outlined in the scoring scheme. Animals in the treated groups had distinctly less severe disease with few exceptions. Differences between animals treated with the wild type (n=11) and aglycosyl (n=12) forms of muMR1 were minimal although composite disease scores for the wild-type forms were slightly lower. FIG. 8 shows the group composite histology scores for the kidneys. Of the animals treated with aglycosyl muMR1, most had mild, early changes in glomeruli with little or no significant tubulo-interstital changes. All animals treated with muMR1 (n=11) had mild early changes and no significant tubulo-interstital changes. From these results, it is clear that both the muMR1 and the aglycosyl muMR1 are effective at preventing the histological changes characteristic of lupus nephritis.

The degree of lymphoid expansion in B and T cell areas was evaluated by histology for each spleen of the treated animals. Identification of secondary follicles was difficult due to artifacts associated with frozen section preparation. No apparent correlation between degree of splenic lymphoid area expansion and renal disease scores was evident. However, the degree of periarteriolar lymphocyte sheath (PALS) expansion appeared to be distinctly greater in isotype control and aglycosyl muMR1-treated animals, as compared to animals treated with muMR1 (data not shown) .

### Analysts of Renal Function

To assess renal function, the proteinuria (PU) levels, as well as serum creatinine and blood urea nitrogen (BUN) measurements of each animal were measured. Both of the chimeric muMR1 mAbs delayed the onset of severe nephritis in SNF1 animals, as measured by the content of protein in the urine. When compared to controls animals, anti-CD154 treated mice had lower PU values at time points between 6 and 9 months (FIG. 9). However, at -10 months of age all groups had PU values indicative of kidney failure (FIG. 9). These results suggest that anti-CD154 treatment delays the onset of proteinuria.

FIG. 11 shows the levels of creatinine in the serum of SNF1 mice, and FIG. 10 shows the levels of BUN in the serum of SNF1 mice. Animals in the control group had elevated serum creatinine and BUN levels in the range of from 7.25 - 10.5 months of age. By contrast, the mice treated with the glycosylated muMR1 remained stable throughout the entire study, with no substantial rise or fall in either serum creatinine or BUN. Aglycosyl muMR1 treated animals maintained normal levels of serum creatinine until -9 months of age, when a slight increase was observed. Similarly, BUN levels in this group became elevated at 11 months of age.

### Survival Assessment

Anti-CD154 mAb treatment prolonged survival of SNF1, mice. At 11 months of age, greater than 90% of treated mice were alive compared to 56% controls. By 14 months, all control mice were dead yet 86% and 75% of muMR1 and aglycosyl muMR1 mice were still alive, respectively. (data not shown)

Collectively, these experiments demonstrate that long-term treatment of nephritic SNF1 mice with either muMR1 or aglycosyl muMRl prolonged survival, decreased autoantibody production and delayed the development of renal pathology. The slightly different results achieved with glycosylated and aglycosyl muMRl indicate a minor role of Fc glycosylation on the efficacy of anti-CD154 mAbs in lupus. Thus, aglycosyl anti-CD154 mAb represents an effective treatment for lupus nephritis.

### METHODS - Example 3

1. Mice. BALB/c, SWR and NZB mice were purchased from The Jackson Laboratory (Bar Harbor, ME). (SWR x NZB)F1 (SNF1) hybrids were bred in the animal facility at Biogen under conventional barrier conditions. Female SNF1 mice were used for all lupus studies. BALB/c mice were used for pharmacokinetic ("PK") studies.

2. Antibodies. The MR1 hybridoma (ATCC #CRL-2580), which produces Armenian hamster anti-mouse CD154 mAb, was purchased from the American Type Culture Collection (Rockville, MD).

3. Treatment Protocol. All injections were given by intraperitoneal route. The lupus study consisted of a control group of SNF1 mice that received PI.17 muIgG2a and treated SNF1 groups that received one of the chimeric anti-CD154 mAbs. A single dose of 500 µg of mAb once per week was given for the first six weeks, followed by a single injection of 500 µg monthly until death of the animal or termination of the study. Studies began when animals were -5.5 months of age and serum samples were collected monthly. For the PK study, BALB/c mice received a single 100 µg dose of muMR1 or aglycosyl muMR1 and blood samples were collected after 4 hours and on days 1, 2, 4, 7, 9, 11 and 14.

4. ELISA Assays. For detecting the chimeric MR1 mAbs in serum, NUNC Maxisorp plates were coated with 5 µg/ml of recombinant soluble murine CD154 overnight at 4°C. The next day the plates were blocked, diluted sera added, followed by horseradish peroxidase conjugated anti-mouse IgG2a (Southern Biotech) and development with TMB substrate. The reaction was stopped with 2N sulfuric acid and plates were read at 450 nm on a Spectramax plate reader (Molecular Devices, CA). Anti-single stranded DNA (ssDNA) and anti-double stranded ("dsDNA") ELISAs were performed using NUNC MaxiSorp plates. Plates were coated overnight at 4°C with 10 µg/ml methylated BSA (Calbiochem Corp, La Jolla, CA) and then with 5 µg/ml grade I calf thymus DNA (SIGMA, St. Louis, MO) for two hours at 25°C. The calf thymus DNA was sheared by sonication and then digested with SI nuclease before use. For the anti-ssDNA assay, the DNA was boiled for 10 min and chilled on ice before use. After blocking, serial dilutions of serum samples were added and incubated at room temperature for 2 hours. Autoantibodies were detected with goat anti-mouse IgG-Alkaline Phosphatase (SIGMA, ST. LOUIS, MO) and developed with p-nitrophenyl phosphate (SIGMA, ST. LOUIS, MO) in 1 M diethanolamine buffer. Plates were read at 405 nm, and standard curves were obtained by using known quantities of anti-DNA mAb 205 or mAb 5c6, which are specific for both ss- and dsDNA. Anti-DNA titers were defined as the reciprocal dilution at 0.1 OD units over background.

5. Histology. Kidney and spleen cryosections and formalin fixed paraffin embedded tissues were stained with hematoxylin-eosin ("H&E") for inflammatory infiltration. Kidneys were also stained with Masson Trichrome for fibrosis and Periodic Acid Schiff stain ("PAS") for basement membrane thickening. The stained tissue sections were scored by a veterinary pathologist. The overall score for histopathologic grading of lupus nephritis was based on glomerular, interstitial, and tubular changes. The grades 0 to 4⁺ are based on percent involvement of the structure being examined (i.e., glomeruli, vessels, etc.) and are as follows: 0, no significant lesions; 1⁺, 1% -30% of architecture affected; 2⁺, 30% - 60% of architecture affected; 3⁺, >60% of architecture affected to some degree; 4⁺, >60% of architecture severely affected.

6. Analysis of Urine and Serum. The urine of each mouse was monitored weekly with Albustix (Bayer Corp., Tarrytown, NY) to measure proteinuria ("PU"). Proteinuria level is scored as follows: 0.5⁺, 15 to 30 mg/dl; 1⁺, 30 mg/dl; 2⁺, 100 mg/dl; 3⁺, 300 mg/dl; 4⁺, >2000 mg/dl. Creatinine and blood urea nitrogen (BUN) were measured in serum on a COBAS Chemistry Analyzer (Roche) intermittently throughout the study to provide measures of renal function in addition to PU.

### Example 4: Aglycosyl muMR1 Antibody Inhibits Experimental Autoimmune Encephalomyelitis (EAE)

Blockade of CD40 ligand at the time of immunization has been shown to suppress the development of EAE [Samoilova, 1997]. In this example, muMR1 and aglycosyl muMR1 antibodies were analyzed for their ability to inhibit EAE. This example also evaluated whether the inhibition of EAE by anti-CD154 mAb was associated with an active suppression mechanism and to what extent it was mediated by a mechanism that is dependent on Fc-dependent interactions of the antibody.

The results of this example demonstrate that aglycosyl muMR1 mAb is as effective an inhibitor of EAE as the wild-type, glycosylated hamster MR1 mAb in blocking the development of clinical disease. More specifically, all MR1 mAbs completely inhibited development of disease, as assessed by mean maximum clinical score and mean severity of disease, with the exception of one mouse in the hamster MR1-treated group. These results suggest that the mechanism underlying protection against EAE by anti-CD154 mAbs does not appear to involve the induction of T regulatory. They also show that Fc-dependent effector functions of the mAb do not play a major role with respect to clinical efficacy but appear to contribute to the underlying mechanism of inhibition in the autoimmune setting of EAE.

### Inhibition of Clinical EAE with Glycosylated muMR1

Figure 12 demonstrates that mice treated with muMR1 did not develop symptoms of disease after primary peptide immunization, as compared with mice treated with the isotype control P1.17. The muMR1 treated animals also did not develop clinical symptoms during the follow-up period of 80 days (data not shown). When mice were re-immunized with PLP139-151 emulsified in complete Freunds' adjuvant, there was an increased severity of clinical symptoms in P1.17-treated animals. Mice that had been treated on days 0, 2 and 4 with muMR1 developed EAE after re-challenge, with a severity that equalled the first phase of EAE in P1.17-treated mice. These results demonstrate that anti-CD154 mAb treatment did not result in active suppression. We also studied whether the transfer of 20 x 10⁶ spleen cells, collected from mice 1 or 3 weeks after EAE-induction and treatment with muMR1, would render naive recipient mice resistant to subsequent active EAE induction. This was not the case (data not shown).

### Inhibition of Clinical EAE with Aglycosyl muMR1

Figure 13 and Table 3 demonstrate that both muMR1 and aglycosyl muMR1 mAbs were effective in inhibiting clinical signs of EAE during the entire follow-up period, when administered as 3 dosages of 200 µg, as compared to the control Ig P1.17. In this regard, the muMR1 and aglycosyl muMR1 antibodies were equally effective as hamster MR1 (data not shown). Administration of lower dosages of these antibodies did not reveal major differences between the antibodies, with respect to their ability to inhibit EAE.

### Inhibition of CNS Inflammatory Infiltrates

To assess whether the antibodies differed in inhibition of inflammatory infiltrates within the central nervous system ("CNS"), separate groups of 4 to 5 mice, treated with different amounts of antibody (muMR1 and algycosyl muMR1, or 3 dosages of 200 µg and P1.17 control Ig), were sacrificed on day 16, at the peak of disease activity in mice treated with the isotype control antibody.

**Table 3: Glycosylation is not required for the inhibitory effect of anti-CD154**

| **Antibody** | **Dosage (µg)** | **N** | **Incidence** | **Mean maximal score ± SD¹** | **Mean cumulative score ± SD¹** |
|---|---|---|---|---|---|
| **P1.17** | 3 x 200 | 14 | 13 | 2.4 ± 0.8 | 32.1 ± 32.5 |
| **muMR1** | 3 x 200 | 13 | 0 | 0 ± 0^{§} | 0 ± 0^{#} |
| **muMR1** | 3 x 75 | 6 | 0 | 0 ± 0^{§} | 0.8 ± 1.2^{# #} |
| **muMR1** | 3 x 25 | 6 | 3 | 1.4 ± 1.6^{§§} | 39.3 ± 53.1 |
| **aglycosyl MR1** | 3 x 200 | 14 | 0 | 0 ± 0^{§} | 0 ± 0^{#} |
| **aglycosyl MR1** | 3 x 75 | 6 | 1 | 0.6 ± 1. 4^{§} | 19.7 ± 47.9 |
| **aglycosyl MR1** | 3 x 25 | 6 | 2 | 0.8 ± 1.3^{§} | 9 ± 17.4* |

| | | | | | |
|---|---|---|---|---|---|
| 1. Development of disease is displayed in Figure 1. For each individual mouse the maximal disability score and the cumulative score during the entire monitoring period was assessed separately before calculating the group means. § p < 0.0005, as compared to P1.17 treated mice ; §§ p<0.05, as compared to P1.17 treated mice # p = 0.002, as compared to P1.17 treated mice ; # # p<0.02, as compared to P1.17 treated mice * p=0.05, as compared to 25 µg muMR1 | | | | | |

As shown in Table 4, the antibodies showed no difference with respect to their ability to suppress the development of inflammatory infiltrates within the CNS during the early phase of disease development. Because it was uncertain whether the mice would develop sub-clinical activity in the absence of signs of EAE, CNS-tissues from 6 to 14 mice per group were analyzed at the endpoint of this study (day 58).

In contrast to mice treated with 200 µg muMR1, both P1.17-treated mice and mice treated with 200 µg aglyMR1 showed evidence of mild inflammatory infiltrates (Table 5) that were predominantly localized in the cerebellum. However, treatment with lower amounts of the antibodies revealed that the aglyMR1 was similar if not somewhat more protective than its glycosylated form. These results demonstrate that both antibodies equally inhibit the development of clinical EAE.

### METHODS - Example 4

1. Induction of EAE. Female SJL mice (10-12 weeks of age, Harlan) were immunized subcutaneously with 50 µg PLP139-151 emulsified in Complete Freunds' Adjuvant (Difco, Detroit, MI). Three days later, mice were injected with 109 heat-killed B. pertussis organisms (RIVM, Bilthoven, The Netherlands) intravenously. Development of EAE was monitored by daily assessment of body weight and a disability score. This score ranges from 0: no symptoms, 0.5: partial loss of tail tonus, 1: complete loss of tail tonus, 2: limb weakness, 2.5:

**Table 4: Effect of anti-CD154 on CNS infiltration (day 16)**

| **Antibody** | **Dosage (µg)** | **Number of animals with infiltrates** | | | |
|---|---|---|---|---|---|
| | | **None** | **Sporadic** | **Moderate** | **Severe** |
| **P1.17** | 3 x 200 | 0 | 3 | 1 | 1 |
| **muMR1** | 3 x 200 | 3 | 1 | 0 | 0 |
| **muMR1** | 3 x 75 | 3 | 2 | 0 | 0 |
| **muMR1** | 3 x 25 | 1 | 1 | 3 | 0 |
| **aglycosyl MR1** | 3 x 200 | 5 | 0 | 0 | 0 |
| **aglycosyl MR1** | 3 x 75 | 4 | 1 | 0 | 0 |
| **aglycosyl MR1** | 3 x 25 | 2 | 1 | 2 | 0 |

**Table 5: Effect of anti-CD154 on CNS infiltration (Day 58)**

| **Antibody** | **Dosage (µg)** | **Number of Animals With Infiltrates** | | | |
|---|---|---|---|---|---|
| | | **None** | **Sporadic** | **Moderate** | **Severe** |
| **P1.17 isotype** | 200 | 3 | 8 | 3 | 0 |
| **muMR** | 200 | 12 | 1 | 0 | 0 |
| **muMR** | 75 | 3 | 3 | 0 | 0 |
| **muMR** | 25 | 0 | 5 | 1 | 0 |
| **aglycosyl MR1** | 200 | 8 | 5 | 1 | 0 |
| **aglycosyl MR1** | 75 | 4 | 2 | 0 | 0 |
| **aglycosyl MR1** | 25 | 4 | 2 | 0 | 0 |

partial paresis, 3: complete paralysis of hind limbs, 3.5: complete paralysis from diaphragm and hind limbs, incontinence, 4: moribund, to 5: death due to EAE.

2. Generation of Antibodies. The variable domains of the heavy and light chains of the hamster anti-mouse CD154 mAb MR1 were cloned by RT-PCR from total RNA from the hybridoma. Expression vectors for hamster/mouse chimeric mAb were constructed by engineering murine IgG2a or murine kappa constant region cDNAs (derived from full-length cDNA clones of the heavy and light chains from the anti-human CD154 mAb - i.e., glycosylated hu5c8) onto the variable domains of the heavy and light chain, respectively, using standard recombinant DNA techniques. Transiently expressed chimeric MR1 mAb, designated muMR1, was demonstrated to recapitulate the CD154 binding properties of the hamster mAb by flow cytometry and immunoprecipitation.

The aglycosyl chimeric MR1, designated aglyMR1, was constructed by site-directed mutagenesis of the heavy chain to change the asparagine residue in the Fc's N-linked glycosylation site (N297 in Kabat EU nomenclature) to a glutamine residue. Stable expression vectors containing CMV-IE promoter-driven tandem transcription cassettes for the immunoglobulin light and heavy chains and a glutamine synthetase gene as a selectable marker were constructed for both muMR1 and agly muMR1 IgG2a, kappa mAbs. The expression vectors were transfected into NS0 cells and stable clones were isolated by selection in glutamine-free medium.

MuMR1 and aglyMR1 were affinity purified from bioreactor cell supernatants on Protein A Sepharose followed by size exclusion chromatography on Sephacryl 300 to remove aggregates. Chromatography resins were purchased from Amersham Pharmacia Biotech (Piscataway, NJ). The mAbs were shown to be >95% pure by SDS-PAGE and endotoxin analysis ensured safeness of these reagents for in vivo use. MuMR1 and aglyMR1 were found to have the same relative affinity for cell surface muCD154 in vitro assays and the same pharmacokinetic half-life in vivo in BALB/c mice (data not shown). The murine IgG2a isotype control mAb, P1.17 (ATCC# TIB-10), was Protein A purified from ascites at Protos Immunoresearch (Burlingame, CA) under contract by Biogen.

3. Delivery of anti-CD154 Antibodies. On Days 0, 2 and 4, each mouse received 200 µl PBS i.p., that also contained the following: Group 1 = PBS (control); Group 2 = 200 µg muMR1; Group 3 - 200 µg Hamster Ig (Ig control); Group 4 = 200 µg murinized MR1; Group 5 = 200 µg P1.17 IgG2a control; Group 6 = 200 µg aglycosyl muMR1.

In some experiments, mice were re-immunized on day 80 with 50 µg PLP139-151 emulsified in Complete Freunds' Adjuvant.

4. Disease Assessment. Mice were weighed daily and monitored for clinical activity during a period of 56 days, according to the following scoring system: 0 = no symptoms; 0.5 = partial loss of tail tonus; 1 = complete loss of tail tonus; 2 = limb weakness; 2.5 = partial paresis; 3 = complete paralysis of hind limbs; 3.5 = complete paralysis from diaphragm and hind limbs, incontinence; 4 = moribund; and 5 = death due to EAE.

5. Histology. Brain tissue and spinal cord of each individual mouse was fixated in 10 % formalin and embedded in paraffin. From each individual mouse, three to six spinal cord sections (4 µm) and six brain sections (each comprising cerebellum, cerebrum, brain stem, and subarachnoid space) separated by 100 µm were analyzed with respect to the extent of inflammatory infiltrates after staining with hematoxylin.

Each individual section was scored according to the following scale: 0 = no infiltrates; 1 = sporadic, mild perivascular infiltration (less than two inflammatory lesions per section); 2 = multi-focal, mild perivascular infiltration; 4 = multi-focal, severe perivascular infiltration accompanied by spreading into the parenchyma. On the basis of the average of all sections, mice were categorized as having no, sporadic, moderate or severe infiltration.

6. Statistical Analysis. Results were analyzed by One-Way ANOVA, followed by post-hoc analysis using the LSD-test. P-values < 0.05 were regarded significant.

### Example 5: Aglycosyl hu5c8 Antibody Inhibits Islet Cell Transplant

Previous studies have shown that rhesus monkeys, treated with a 20 mg/kg induction/maintenance-dosing regime of glycosylated hu5c8 maintained renal allograft function and none experienced a rejection episode during the six-month dosing period, while untreated monkeys that received renal allografts promptly rejected their transplants within eight days [Kirk, 1999]. In a related study by Kirk's research group, it was demonstrated that aglycosyl hu5c8, was ineffective for the treatment of transplant rejection.

In striking contrast to Kirk's findings, the results of this invention demonstrate that an aglycosylated form of the hu5c8 mAb may in fact be effective for the treatment of transplant rejection in other settings.

### Islet Cell Allograft Transplant in Rhesus Monkeys.

It has previously been demonstrated that glycosylated hu5c8 enables islet engraftment and maintains allograft survival [Kenyon, 1999].

Four rhesus monkeys treated with a 20 mg/kg induction/maintenance regimen achieved insulin independence for >213, >255, >269, and >341 days post-transplant. The results of this study showed that the untreated control monkeys that received islet allografts exhibited acute rejection by day 8, as was evidenced by persistent hyperglycemia and lack of c-peptide production (a product of endogenous insulin production) by day 11-14 (data not shown) .

In contrast to the untreated control animals, FIG. 14 shows the successful treatment of one of two rhesus monkeys treated with an induction/maintenance regimen of aglycosyl hu5c8 (described above). Although both monkeys experienced hyperglycemia and initial rejection on day 7, when the monkeys were treated with insulin and aglycosyl hu5c8 treatment was continued, one of the two monkeys exhibited partial function of the allograft as measured by the presence of c-peptide at day 28 (open diamond), and was maintained through day 45.

These results suggest that aglycosylated anti-CD154 mAbs are useful in treatment regimens in a transplant setting. However, because the immune response during transplant is such a strong response, i.e., involves humoral, cellular and inflammatory immune responses, it is possible that aglycosyl anti-CD154 antibodies may be most effective when delivered in combination with an immunosuppressive or immunomodulatory compound. For example, an agent that interrupts T cell costimulatory signaling via CD28; an agent that interrupts calcineurin signaling, a corticosteroid, an anti-proliferative agent, or other antibody that specifically binds to a protein expressed on the surface of immune cells, including but not limited to CD45, CD2, IL2R, CD4, CD8 and RANK FcR, B7, CTLA4, TNF, LTβ, and VLA-4.

### METHODS - Example 5

1. Islet Cell Allograft Transplant. These studies were performed as described previously [Kenyon, 1999]. In brief, alloreactive donor-recipient pairs of rhesus monkeys were chosen based on positive mixed lymphocyte culture reactivity. Recipients underwent complete pancreatectomy and intraportal, allogeneic islet transplantation on Day 0.

2. Antibody Treatment. Dosing was performed using an induction consisting of 20 mg/kg on Days -1, 0, 3, 10 and 18 and maintenance consisted of one 20 mg/kg dose per month starting at day 28.

3. Graft Function Assessment. Islet graft function was monitored daily through fasting and postprandial blood glucose levels. Islet failure (primary nonfunction or acute rejection) was defined as an absence of fasting and stimulated c-peptide production (an endogeneous insulin product). Primary nonfunction was defined as failure of the transplanted tissue to function in the immediate post-transplant period and was characterized by persistent hyperglycemia and unstable glycemic control. Acute rejection episodes were defined as fasting glucose >100 mg/dL and a postprandial blood glucose >150-175 mg/dL. Overall graft function was evaluated by monitoring the amount of exogenous insulin required to maintain normal blood glucose levels.

### Example 6: Use of Aglycosylated hu5c8 (Anti-CD154) Antibody to Prevent Anti-CD154-Mediated T-Cell Alloreactivity

Monoclonal antibodies targeting CD154 on T cells have been shown to partially prevent in vitro as well as in vivo alloreactivity. However, it is not clear whether the suppressive activity of anti-CD154 mAb depends on the blockade of stimulatory CD40-CD154 interactions or, alternatively, on the direct delivery of inhibitory signals through CD154.

To assess the effects of blockade of stimulatory CD40-CD154 interactions as opposed to stimulation of CD154 (i.e., direct delivery of inhibitory signals through CD154) on human T cell alloreactivity in vitro, unfractionated PBMC, or purified CD4+ T cells, were cocultured with allogeneic, HLA-mismatched, stimulator cells (CD40 positive cells) in the presence of a humanized anti-CD154 mAb (hu5c8, Biogen Inc., MA, USA), either soluble or immobilized to the culture wells. Most blocking experiments were performed with a genetically engineered variant of soluble anti-CD154 (aglycosylated hu5c8) that has reduced Fc-effector function and therefore may have a reduced ability to cross-link CD154. The aglycosylated hu5c8 used in this example is the same as the aglycosyl hu5c8 mAb described throughout this specification. See, e.g., Example 1, ¶ 15 and ¶ 83, supra. Soluble but not coated anti-CD154 antibodies inhibited allogeneic T cell proliferation in primary mixed lymphocyte culture ("MLC") (40±23% vs 3±18% inhibition, n=4). Similarly, proliferation of alloantigen-specific T lymphocytes in secondary MLC (n=5 experiments) was suppressed by priming with CD154 blockade (with soluble antibodies), whereas it was increased by CD154 stimulation (coated antibodies). Moreover, stimulation by coated anti-CD154 antibodies strongly enhanced the generation of alloantigen-specific CTL effectors, while CTLs were not affected by CD154 blockade.

To test whether the stimulatory activity of anti-CD154 required B7-CD28 costimulatory interactions, MLC were performed in the presence of CTLA4-Ig, a molecule that prevents binding of B7 molecules to CD28. Priming in the presence of CTLA4-Ig suppressed the proliferation of alloantigen-specific T lymphocytes in primary and secondary MLC by, respectively, 75±14% (n=3) and 64±28% (n=6) and inhibited CTL generation by 48±23% (n=2). Signaling of coated anti-CD154 antibodies significantly increased the residual CD28-independent proliferation of alloantigen-specific T cells both in primary (by 58±0.6%, n=2) and secondary (by 61±49%, n=6) MLC, although it did not completely abrogate CTLA4-Ig-induced inhibition. However, the inhibiting effect of CTLA4-Ig on the generation of CTL was abrogated by CD154 stimulation (via coated anti-CD154 antibodies). Expression of CD25, HLA-DR and CD95 on alloreactive T cells was strongly increased by stimulation of CD154 (n=2), as compared to control cultures with or without CTLA4-Ig.

Our data show that anti-CD154 antibodies can enhance, rather than block, T cell alloreactivity, when immobilized to the culture plate. Blockade of CD154 by soluble aglycosylated hu5c8 mAb was unable to enhance T cell activation in vitro and may therefore be advantageous in vivo and, based on these findings, could be effective, in transplant experimental models, to reduce alloantigens T cell responses in vivo, either alone, or in combination with molecules blocking other costimulatory pathways.

### REFERENCES

1. Salazar-Fontana, L. I., and B. E. TBierer 2001. Curr. Opin. Hemat. 8:5.
2. Foy, T. M., A. Aruffo, J. Bajorath, J. E. Buhlmann, and R. J. Noelle 1996. Ann. Rev. Immunol. 14:591.
3. Burkly, L. C. 2001. In Adv. Exp. Med. Bio., Vol. 489. D. M. Monroe, U. Hedner, M. R. Hoffman, C. Negrier, G. F. Savidge, and G. C. I. White, eds. Kluwer Academic/Plenum Publishers, p. 135.
4. Nose, M., and H. Wigzell 1983. Proc. Nat. Aca. of Sci. USA 80:6632.
5. Leatherbarrow, R. J., T. W. Rademacher, R. A. Dwek, J. M. Woof, A. Clark, D. R. Burton, N. Richardson, and A. Feinstein 1985. Mol. Immunol. 22:407.
6. Tao, M. H., and S. L. Morrison 1989. J. Immunol. 143:2595.
7. Lund, J., T. Tanaka, N. Takahashi, G. Sarmay, Y. Arata, and R. Jefferis 1990. Mol. Immunol. 27:1145.
8. Dorai, H., B. M. Mueller, R. A. Reisfeld, and S. D. Gillies 1991. Hybridoma 10:211.
9. Hand, P. H., B. Calvo, D. Milenic, T. Yokota, M. Finch, P. Snoy, K. Garmestani, O. Gansow, J. Schlom, and S. V. Kashmiri 1992. Cancer. Immunol. Immunother. 35:165.
10. Leader, K. A., B. M. Kumpel, A. G. Hadley, and B. A. Bradley 1991. Immunology 72:481.
11. Pound, J. D., J. Lund, and R. Jefferis 1993. Mol. Immunol. 30:233.
12. Boyd, P. N., A. C. Lines, and A. K. Patel 1995. Mol. Immunol. 32:1311.
13. Isaacs, J. D., M. R. Clark, J. Greenwood, and H. Waldmann. 1992. J. Immunol. 148:3062.
14. Friend, P. J., G. Hale, L. Chatenoud, P. Rebello, J. Bradley, S. Thiru, J. M. Phillips, and H. Waldmann 1999. Transplantation 68:1632.
15. Hobbs, S. M., L. E. Jackson, and J. Hoadley. 1992. Mol. Immunol . 29 : 949.
16. Morrison et al. 1984 Proc. Natl. Acad. Sci. USA 81(21):6851-5.
17. Sharon et al. 1984. Nature 309(5966) :364-7.
18. Takeda et al. 1985. Nature 314(6010):452-4.
19. Riechmann et al. 1988. Nature 332(6162):323-7.
20. Co et al. 1991. Nature 351 (6326) :501-2.
21. Jones et al. 1986. Nature 321:522-525.
22. Riechmann et al. 1988. Nature 332:323-327.
23. Verhoeyen et al. 1988. Science 239:1534-1536.
24. Queen et al. 1989. Proc. Nat. Acad. Sci. USA 86:10029.
25. Orlandi et al. 1989. Proc. Natl. Acad. Sci. USA 86:3833.
26. Co et al. 1991. Proc. Nat. Acad. Sci. USA 88:2869-2873.
27. Tempest 1991. Biotechnology 9: 266-271.
28. Boerner et al. 1991. J. Immunol. 147:86-95.
29. Persson et al. 1991. Proc. Nat. Acad. Sci. USA 88: 2432-2436.
30. Huang and Stollar 1991. J. Immunol. Methods 141: 227-236.
31. Green et al. 1994. Nature Genetics 7:13-21.
32. Mendez et al. 1997. Nature Genetics 15(2) :146-56.
33. Williams, R.C. et al. 1973. J. Immunol. 111(6): 1690-8.
34. Winkelhake & Nicolson 1976. J. Biol. Chem. 251(4): 1074-80.
35. Nose, M., and H. Wigzell. 1983. Proc. Natl. Acad. Sci. USA 80:6632.
36. Harlow et al. (eds.) 1998. Antibodies: A Laboratory Manual, Cold Spring Harbor Laboratory.
37. Coligan et al. (eds.) 2001. Current Protocols in Immunology, John Wiley & Sons, Inc.
38. Zola 2000. Monoclonal Antibodies: Preparation and Use of Monoclonal Antibodies and Engineered Antibody Derivatives (Basics: From Background to Bench), Springer Verlag.
39. Howard et al. (eds.) 2000. Basic Methods in Antibody Production and Characterization, CRC Press.
40. Davis (ed.) 1995. Monoclonal Antibody Protocols, Vol. 45, Humana Press.
41. Delves (ed.) 1997. Antibody Production: Essential Techniques, John Wiley & Son Ltd.
42. Kenney 1997. Antibody Solution: An Antibody Methods Manual, Chapman & Hall.
43. Pollock et al. 1999. J. Immunol. Meth. 231(1-2):147-57.
44. Abuchowski et al. 1981. In: "Enzymes as Drugs".
45. Holcenberg et al. (ed.) 1981. Wiley-Interscience, New York, NY, 367-383 (1981).
46. Anderson, W.F. 1992. Human Gene Therapy. Science 256:808-813.
47. Newmark et al. 1982. J. Appl. Biochem. 4:185-189.
48. Katre et al. 1987. Proc. Natl. Acad. Sci. USA 84:1487-1491.
49. Gallin 1989. Fundamental Immunology, Chapter 26, Raven Press, 2d Ed., pp. 721-733, New York.
50. Samoilova, E.B. et al. 1997. J. Mol. Med., 75:603, 1997.
51. Kirk, A. D., L. C. Burkly, D. S. Batty, R. E. Baumgartner, J. D. Berning, K. Buchanan, J. H. Fechner, Jr., R. L. Germond, R. L. Kampen, N. B. Patterson, S. J. Swanson, D. K. Tadaki, C. N. TenHoor, L. White, S. J. Knechtle, and D. M. Harlan 1999. Nat. Med. 5:686.
52. Kenyon, N. S., M. Chatzipetrou, M. Masetti, A. Ranuncoli, M. Oliveira, J. L. Wagner, A. D. Kirk, D. M. Harlan, L. C. Burkly, and C. Ricordi. 1999. Proc. Natl. Acad. Sci. USA 96:8132.

## Claims

1. An aglycosyl anti-CD154 antibody or antibody derivative, **characterized by** a modification at the conserved N-linked site in the C_{H}2 domains of the Fc portion of said antibody.

2. The aglycosyl anti-CD154 antibody or antibody derivative according to claim 1, wherein the modification comprises a mutation in the heavy chain glycosylation site, wherein the mutation prevents glycosylation at the site.

3. The aglycosyl anti-CD154 antibody or antibody derivative according to claim 2, wherein the modification comprises a mutation of N298Q (N297 using EU Kabat numbering).

4. The aglycosyl anti-CD154 antibody or antibody derivative according to claim 1, wherein the modification comprises the removal of the C_{H}2 domain glycans.

5. The aglycosyl anti-CD154 antibody or antibody derivative according to claim 1, wherein the modification comprises prevention of glycosylation at the C_{H}2 domain.

6. The aglycosyl anti-CD154 antibody or antibody derivative according to any one of claims 1 to 5, wherein said aglycosyl anti-CD154 antibody or antibody derivative does not bind to an effector receptor.

7. The aglycosyl anti-CD154 antibody or antibody derivative according to any one of claims 1 to 5, wherein said aglycosyl anti-CD154 antibody or antibody derivative does not cause thrombosis.

8. The aglycosyl anti-CD154 antibody or antibody derivative according to any one of claims 1 to 5, wherein said antibody is selected from the group consisting of: a monoclonal antibody, a polyclonal antibody, a murine antibody, a chimeric antibody, a primatized antibody, a humanized antibody, and a fully human antibody.

9. The aglycosyl anti-CD154 antibody or antibody derivative according to any one of claims 1 to 5, wherein said antibody is selected from the group consisting of: a multimeric antibody, a heterodimeric antibody, a hemidimeric antibody, a tetravalent antibody, and a bispecific antibody.

10. The aglycosyl anti-CD154 antibody or antibody derivative according to any one of claims 1 to 5, wherein said antibody is aglycosyl hu5c8 produced by the cell line having ATCC Accession No. PTA-4931.

11. The aglycosyl anti-CD154 antibody or antibody derivative according to any one of claims 1 to 5, wherein said antibody or antibody derivative is labeled with a detectable marker.

12. The aglycosyl anti-CD154 antibody or antibody derivative according to claim 11, wherein the detectable marker is a radioactive isotope, enzyme, dye or biotin.

13. The aglycosyl anti-CD154 antibody or antibody derivative according to any one of claims 1 to 5, wherein said antibody or antibody derivative is conjugated to a therapeutic agent.

14. The aglycosyl anti-CD154 antibody or antibody derivative according to claim 13, wherein said therapeutic agent is a radioisotope, radionuclide, toxin, toxoid or chemotherapeutic agent.

15. The aglycosyl anti-CD154 antibody or antibody derivative according to any one of claims 1 to 5, wherein said antibody or antibody derivative is conjugated to an imaging agent.

16. The aglycosyl anti-CD154 antibody or antibody derivative according to claim 15, wherein the imaging agent is a labeling moiety.

17. The aglycosyl anti-CD154 antibody or antibody derivative according to claim 15, wherein the imaging agent is a biotin, a fluorescent moiety, a radioactive moiety, a histidine tag, or a peptide tag.

18. A pharmaceutical composition comprising the aglycosyl anti-CD154 antibody or antibody derivative according to any one of claims 1 to 5.

19. The pharmaceutical composition according to claim 18, further comprising a pharmaceutically acceptable carrier.

20. The pharmaceutical composition according to claim 18, further comprising an immunosuppressive or immunomodulatory compound.

21. A cell line producing the aglycosyl anti-CD154 antibody or antibody derivative according to any one of claims 1 to 5.

22. The cell line according to claim 21, wherein the cell line produces the aglycosyl hu5c8 (ATCC Accession No. PTA-4931).

23. Use of an aglycosyl anti-CD154 antibody or an aglycosyl anti-CD154 antibody derivative according to any one of claims 1 to 5, or a pharmaceutical composition comprising said antibody or antibody derivative, for the manufacture of a medicament for inhibiting an immune response, an inflammatory response, transplant rejection, graft-vs-host disease, an allergic response, an autoimmune response, fibrosis, viral infection of the T cells by the HTLV I virus, gastrointestinal disease, vascular disease, or proliferation of T cell tumor cells, in a subject.

24. The use according to claim 23, wherein the aglycosyl anti-CD154 antibody, antibody derivative or pharmaceutical composition inhibits binding of CD154 to CD40.

25. The use according to claim 23, wherein the aglycosyl anti-CD154 antibody, antibody derivative or pharmaceutical composition is capable of specifically binding to a protein that is specifically recognized by aglycosyl hu5c8, which is produced by the cell line having ATCC Accession No. PTA-4931.

26. The use according to claim 23, wherein the aglycosyl anti-CD154 antibody, antibody derivative or pharmaceutical composition specifically binds to the epitope to which aglycosyl hu5c8 (ATCC Accession No. PTA-4931) specifically binds.

27. The use according to claim 23, wherein the inflammatory response is selected from the group consisting of: arthritis, contact dermatitis, hyper-IgE syndrome, inflammatory bowel disease, allergic asthma and idiopathic inflammatory disease.

28. The use according to claim 27, wherein the arthritis is selected from the group consisting of: rheumatoid arthritis, non-rheumatoid inflammatory arthritis, arthritis associated with Lyme disease and inflammatory osteoarthritis.

29. The use according to claim 27, wherein the idiopathic inflammatory disease is selected from the group consisting of: psoriasis and systemic lupus.

30. The use according to claim 23, wherein the transplant rejection involves transplanted heart, kidney, liver, skin, pancreatic islet cells or bone marrow.

31. The use according to claim 23, wherein the allergic response is selected from the group consisting of: hay fever or an allergy to penicillin or other drugs.

32. The use according to claim 23, wherein the autoimmune response derives from an infectious disease.

33. The use according to claim 23, wherein the autoimmune response derives from Reiter's syndrome, spondyloarthritis, Lyme disease, HIV infections, syphilis or tuberculosis.

34. The use according to claim 23, wherein the autoimmune response is selected from the group consisting of: rheumatoid arthritis, Myasthenia gravis, systemic lupus erythematosus, Graves' disease, idiopathic thrombocytopenia purpura, hemolytic anemia, diabetes mellitus, inflammatory bowel disease, Crohn's disease, multiple sclerosis, psoriasis, drug-induced autoimmune diseases, and drug-induced lupus.

35. The use according to claim 23, wherein the fibrosis is selected from the group consisting of: pulmonary fibrosis and fibrotic disease.

36. The use according to claim 35, wherein the pulmonary fibrosis is selected from the group consisting of: pulmonary fibrosis secondary to adult respiratory distress syndrome, drug-induced pulmonary fibrosis, idiopathic pulmonary fibrosis and hypersensitivity pneumonitis.

37. The use according to claim 35, wherein the fibrotic disease is selected from the group consisting of: Hepatitis-C; Hepatitis-B; cirrhosis; cirrhosis of the liver secondary to a toxic insult; cirrhosis of the liver secondary to drugs; cirrhosis of the liver secondary to a viral infection and cirrhosis of the liver secondary to an autoimmune disease.

38. The use according to claim 23, wherein the gastrointestinal disease is selected from the group consisting of: esophageal dysmotility, inflammatory bowel disease and scleroderma.

39. The use according to claim 23, wherein the vascular disease is selected from the group consisting of: atherosclerosis or reperfusion injury.

40. The use according to any one of claims 23 to 39, wherein the aglycosyl anti-CD154 antibody or aglycosyl anti-CD154 antibody derivative is selected from the group consisting of: a monoclonal antibody, a polyclonal antibody, a murine antibody, a chimeric antibody, a primatized antibody, a humanized antibody, and a fully human antibody.

41. The use according to any one of claims 23 to 39, wherein the aglycosyl anti-CD154 antibody or aglycosyl anti-CD154 antibody derivative is selected from the group consisting of: a multimeric antibody, a heterodimeric antibody, a hemidimeric antibody, a tetravalent antibody, and a bispecific antibody.

42. The use according to any one of claims 23 to 39, wherein the aglycosyl anti-CD154 antibody or aglycosyl anti-CD154 antibody derivative or pharmaceutical composition comprising said antibody or antibody derivative is to be administered to said subject by injection intravenously, subcutaneously, intraperitoneally, intramuscularly, intramedullarily, intraventricularly, intraepidurally, intraarterially, intravascularly, intra-articularly, intra-synovially, intrasternally, intrathecally, intrahepatically, intraspinally, intratumorly, intracranially; by an enteral, intrapulmonary, transmucosal, intrauterine, or sublingual route of administration; or locally at sites of inflammation or tumor growth.

43. The use according to any one of claims 23 to 39, wherein the aglycosyl anti-CD154 antibody, aglycosyl anti-CD154 antibody derivative or pharmaceutical composition comprising said antibody or antibody derivative is to be administered to said subject by a route selected from the group consisting of: oral, nasal, ophthalmic, rectal, and topical routes.

44. The use according to claim 43, wherein the aglycosyl anti-CD154 antibody, aglycosyl anti-CD154 antibody derivative or pharmaceutical composition comprising said antibody or antibody derivative is to be administered to said subject orally in the form of capsules, tablets, aqueous suspensions or solutions.

45. The use according to claim 43, wherein the aglycosyl anti-CD154 antibody, aglycosyl anti-CD154 antibody derivative or pharmaceutical composition comprising said antibody or antibody derivative is to be administered to said subject topically by application of a cream, ointment or the like.

46. The use according to claim 43, wherein the aglycosyl anti-CD154 antibody, aglycosyl anti-CD154 antibody derivative or pharmaceutical composition comprising said antibody or antibody derivative is to be administered to said subject by inhalation through the use of a nebulizer, a dry powder inhaler or a metered dose inhaler.

47. The use according to any one of claims 23 to 39, wherein the aglycosyl anti-CD154 antibody, aglycosyl anti-CD154 antibody derivative or pharmaceutical composition comprising said antibody or antibody derivative is to be administered to said subject by sustained release administration.

48. The use according to any one of claims 23 to 39, wherein the aglycosyl anti-CD154 antibody, aglycosyl anti-CD154 antibody derivative or pharmaceutical composition comprising said antibody or antibody derivative is to be administered to said subject along with a therapeutic agent.

49. The use according to any one of claims 23 to 39, wherein the aglycosyl anti-CD154 antibody, aglycosyl anti-CD154 antibody derivative or pharmaceutical composition comprising said antibody or antibody derivative is to be administered to said subject in multiple doses per day.

50. The use according to any one of claims 23 to 39, wherein the aglycosyl anti-CD154 antibody, aglycosyl anti-CD154 antibody derivative or pharmaceutical composition comprising said antibody or antibody derivative is to be administered to said subject repeatedly at intervals ranging from each day to every other month.

51. The use according to any one of claims 23 to 39, wherein the aglycosyl anti-CD154 antibody, aglycosyl anti-CD154 antibody derivative or pharmaceutical composition comprising said antibody or antibody derivative is to be administered to said subject at intervals for as long a time as medically indicated, ranging from days or weeks to the life of the subject.

52. The use according to any one of claims 23 to 39, wherein the aglycosyl anti-CD154 antibody, aglycosyl anti-CD154 antibody derivative or pharmaceutical composition comprising the antibody or antibody derivative is to be administered to the subject with an immunomodulatory or immunosuppressive compound.

53. The use according to claim 52, wherein the immunomodulatory or immunosuppressive compound is selected from the group consisting of:
(a) an agent that interrupts T cell costimulatory signalling via CD28;
(b) an agent that interrupts calcineurin signalling,
(c) a corticosteroid,
(d) an anti-proliferative agent; and
(e) an antibody that specifically binds to a protein expressed on the surface of immune cells, including but not limited to CD45, CD2, IL2R, CD4, CD8 and RANK FcR, B7, CTLA4, TNF, LTβ, and VLA-4.

54. The use according to claim 52, wherein the immunosuppressive or immunomodulatory compound is selected from the group consisting of: tacrolimus, sirolimus, mycophenolate mofetil, mizorubine, deoxyspergualin, brequinar sodium, leflunomide, rapamycin and azaspirane.

55. Use of the aglycosyl anti-CD154 antibody or antibody derivative according to any one of claims 1 to 5 for the manufacture of a composition for use in a method for imaging tumor cells or neoplastic cells in a subject that express a protein that is specifically recognized by aglycosyl hu5c8 produced by the cell line having ATCC Accession No. PTA-4931, said method comprising the steps of:
(a) administering to the subject an effective amount of the composition under conditions permitting the formation of a complex between the antibody or antibody derivative and a protein on the surface of tumor cells or neoplastic cells; and
(b) imaging any antibody/protein complex or antibody derivative/complex formed, thereby imaging any tumor cells or neoplastic cells in the subject.

56. Use of the aglycosyl anti-CD154 antibody or antibody derivative according to any one of claims 1 to 5 for the manufacture of a composition for use in a method for detecting the presence of tumor cells or neoplastic cells in a subject that express a protein that is specifically recognized by aglycosyl hu5c8 produced by the cell line having ATCC Accession No. PTA-4931, said method comprising the steps of:
(a) administering to the subject an effective amount of the composition under conditions permitting the formation of a complex between the antibody or antibody derivative and the protein;
(b) clearing any unbound imaging agent from the subject; and
(c) detecting the presence of any antibody/protein complex or antibody derivative/complex formed, the presence of such complex indicating the presence of tumor cells or neoplastic cells in the subject.

## Patentansprüche

1. Aglycosyl-anti-CD154-Antikörper oder -Antikörperderivat, **gekennzeichnet durch** eine Modifizierung an der konservierten N-gebundenen Stelle in den CH₂-Domänen des Fc-Anteils des Antikörpers.

2. Aglycosyl-anti-CD154-Antikörper oder -Antikörperderivat gemäß Anspruch 1, wobei die Modifizierung eine Mutation in der Glycosylierungsstelle der schweren Kette umfasst, wobei die Mutation eine Glycosylierung an der Stelle verhindert.

3. Aglycosyl-anti-CD154-Antikörper oder -Antikörperderivat gemäß Anspruch 2, wobei die Modifizierung eine Mutation von N298Q (N297 unter Verwendung der EU-Kabat-Numerierung) umfasst.

4. Aglycosyl-anti-CD154-Antikörper oder -Antikörperderivat gemäß Anspruch 1, wobei die Modifizierung die Entfernung der C_{H}2-Domänen-Glycane umfasst.

5. Aglycosyl-anti-CD154-Antikörper oder -Antikörperderivat gemäß Anspruch 1, wobei die Modifizierung die Verhinderung einer Glycosylierung an der C_{H}2-Domäne umfasst.

6. Aglycosyl-anti-CD154-Antikörper oder -Antikörperderivat gemäß einem der Ansprüche 1 bis 5, wobei der bzw. das Aglycosyl-anti-CD154-Antikörper oder -Antikörperderivat nicht an einen Effektorrezeptor binden.

7. Aglycosyl-anti-CD154-Antikörper oder -Antikörperderivat gemäß einem der Ansprüche 1 bis 5, wobei der bzw. das Aglycosyl-anti-CD154-Antikörper oder - Antikörperderivat nicht Thrombose verursachen.

8. Aglycosyl-anti-CD154-Antikörper oder -Antikörperderivat gemäß einem der Ansprüche 1 bis 5, wobei der Antikörper aus der Gruppe ausgewählt ist, welche besteht aus: einem monoklonalen Antikörper, einem polyklonalen Antikörper, einem murinen Antikörper, einem chimären Antikörper, einem primatisierten Antikörper, einem humanisierten Antikörper und einem vollständig humanen Antikörper.

9. Aglycosyl-anti-CD154-Antikörper oder -Antikörperderivat gemäß einem der Ansprüche 1 bis 5, wobei der Antikörper aus der Gruppe ausgewählt ist, welche besteht aus: einem multimeren Antikörper, einem heterodimeren Antikörper, einem hemidimeren Antikörper, einem tetravalenten Antikörper und einem bispezifischen Antikörper.

10. Aglycosyl-anti-CD154-Antikörper oder -Antikörperderivat gemäß einem der Ansprüche 1 bis 5, wobei der Antikörper Aglycosyl hu5c8, welcher durch die Zelllinie mit der ATCC-Zugangsnr. PTA-4931 hergestellt wird, ist.

11. Aglycosyl-anti-CD154-Antikörper oder -Antikörperderivat gemäß einem der Ansprüche 1 bis 5, wobei der Antikörper oder das Antikörperderivat mit einem nachweisbaren Marker markiert sind.

12. Aglycosyl-anti-CD154-Antikörper oder -Antikörperderivat gemäß Anspruch 11, wobei der nachweisbare Marker ein radioaktives Isotop, Enzym, Farbstoff oder Biotin ist.

13. Aglycosyl-anti-CD154-Antikörper oder -Antikörperderivat gemäß einem der Ansprüche 1 bis 5, wobei der Antikörper oder das Antikörperderivat an ein therapeutisches Mittel konjugiert sind.

14. Aglycosyl-anti-CD154-Antikörper oder -Antikörperderivat gemäß Anspruch 13, wobei das therapeutische Mittel ein Radioisotop, Radionuklid, Toxin, Toxoid oder chemotherapeutisches Mittel ist.

15. Aglycosyl-anti-CD154-Antikörper oder -Antikörperderivat gemäß einem der Ansprüche 1 bis 5, wobei der Antikörper oder das Antikörperderivat an ein Mittel zur Bildgebung konjugiert sind.

16. Aglycosyl-anti-CD154-Antikörper oder -Antikörperderivat gemäß Anspruch 15, wobei das Mittel zur Bildgebung eine markierende Einheit ist.

17. Aglycosyl-anti-CD154-Antikörper oder -Antikörperderivat gemäß Anspruch 15, wobei das Mittel zur Bildgebung ein Biotin, eine fluoreszente Einheit, eine radioaktive Einheit, ein Histidin-Tag oder ein Peptid-Tag ist.

18. Pharmazeutische Zusammensetzung, umfassend den bzw. das Aglycosyl-anti-CD154-Antikörper oder -Antikörperderivat gemäß einem der Ansprüche 1 bis 5.

19. Pharmazeutische Zusammensetzung gemäß Anspruch 18, ferner umfassend einen pharmazeutisch verträglichen Träger.

20. Pharmazeutische Zusammensetzung gemäß Anspruch 18, ferner umfassend eine immunsuppressive oder immunomodulatorische Verbindung.

21. Zellinie, welche den bzw. das Aglycosyl-anti-CD 154-Antikörper oder -Antikörperderivat gemäß einem der Ansprüche 1 bis 5 herstellt.

22. Zellinie gemäß Anspruch 21, wobei die Zellinie den Aglycosyl hu5c8 (ATCC-Zugangsnr. PTA-4931) herstellt.

23. Verwendung eines Aglycosyl-anti-CD154-Antikörpers oder eines Aglycosyl-anti-CD154-Antikörperderivats gemäß einem der Ansprüche 1 bis 5 oder einer pharmazeutischen Zusammensetzung, umfassend den Antikörper oder das Antikörperderivat, zur Herstellung eines Medikaments zur Inhibierung einer Immunantwort, einer Entzündungsantwort, Transplantatabstoßung, Transplantat-Wirt-Reaktion, einer allergischen Antwort, einer Autoimmunantwort, Fibrose, viralen Infektion der T-Zellen durch das HTLV I-Virus, gastrointestinalen Erkrankung, vaskulären Erkrankung oder Proliferation von T-Zell-Tumorzellen in einem Empfänger.

24. Verwendung gemäß Anspruch 23, wobei der bzw. das Aglycosyl-anti-CD154-Antikörper, -Antikörperderivat oder die pharmazeutische Zusammensetzung die Bindung von CD154 an CD40 inhibieren.

25. Verwendung gemäß Anspruch 23, wobei der bzw. das Aglycosyl-anti-CD154-Antikörper, -Antikörperderivat oder die pharmazeutische Zusammensetzung in der Lage sind, spezifisch an ein Protein zu binden, welches spezifisch durch Aglycosyl hu5c8, der durch die Zellinie mit der ATCC-Zugangsnr. PTA-4931 hergestellt wird, erkannt wird.

26. Verwendung gemäß Anspruch 23, wobei der bzw. das Aglycosyl-anti-CD154-Antikörper, -Antikörperderivat oder die pharmazeutische Zusammensetzung spezifisch an das Epitop binden, an welches Aglycosyl hu5c8 (ATCC-Zugangsnr. PTA-4931) spezifisch bindet.

27. Verwendung gemäß Anspruch 23, wobei die Entzündungsantwort aus der Gruppe ausgewählt ist, welche besteht aus: Arthritis, Kontaktdermatitis, Hyper-IgE-Syndrom, entzündlicher Darmerkrankung, allergischem Asthma und idiopathischer Entzündungserkrankung.

28. Verwendung gemäß Anspruch 27, wobei die Arthritis aus der Gruppe ausgewählt ist, welche besteht aus: rheumatoider Arthritis, nicht-rheumatoider entzündlicher Arthritis, Arthritis in Zusammenhang mit Lyme-Krankheit und entzündlicher Osteoarthritis.

29. Verwendung gemäß Anspruch 27, wobei die idiopathische Entzündungserkrankung aus der Gruppe ausgewählt ist, welche besteht aus: Psoriasis und systemischem Lupus.

30. Verwendung gemäß Anspruch 23, wobei die Transplantatabstoßung transplantierte bzw. transplantiertes Herz, Niere, Leber, Haut, Pankreasinselzellen oder Knochenmark einbezieht.

31. Verwendung gemäß Anspruch 23, wobei die allergische Antwort aus der Gruppe ausgewählt ist, welche besteht aus: Heuschnupfen oder einer Allergie gegen Penicillin oder andere Arzneistoffe.

32. Verwendung gemäß Anspruch 23, wobei sich die Autoimmunantwort von einer infektiösen Erkrankung ableitet.

33. Verwendung gemäß Anspruch 23, wobei sich die Autoimmunantwort von Reiter-Syndrom, Spondyloarthritis, Lyme-Krankheit, HIV-Infektionen, Syphilis oder Tuberkulose ableitet.

34. Verwendung gemäß Anspruch 23, wobei die Autoimmunantwort aus der Gruppe ausgewählt ist, welche besteht aus: rheumatoider Arthritis, Myasthenia gravis, systemischem Lupus erythematodes, Morbus Basedow (Grave's disease), idiopathischer thrombozytopenischer Purpura, hämolytischer Anämie, Diabetes mellitus, entzündlicher Darmerkrankung, Morbus Crohn, Multipler Sklerose, Psoriasis, Arzneistoff-induzierten Autoimmunerkrankungen und Arzneistoffinduziertem Lupus.

35. Verwendung gemäß Anspruch 23, wobei die Fibrose aus der Gruppe ausgewählt ist, welche besteht aus: Lungenfibrose und fibrotischer Erkrankung.

36. Verwendung gemäß Anspruch 35, wobei die Lungenfibrose aus der Gruppe ausgewählt ist, welche besteht aus: Lungenfibrose, welche als Begleiterscheinung bei Schocklunge auftritt, Arzneistoff-induzierter Lungenfibrose, idiopathischer Lungenfibrose und Hypersensitivitätpneumonitis.

37. Verwendung gemäß Anspruch 35, wobei die fibrotische Erkrankung aus der Gruppe ausgewählt ist, welche besteht aus: Hepatitis C; Hepatitis B; Leberzirrhose, welche als Begleiterscheinung bei einem toxischen Trauma auftritt; Leberzirrhose, welche als Begleiterscheinung bei Arzneistoffen auftritt; Leberzirrhose, welche als Begleiterscheinung bei einer Virusinfektion auftritt, und Leberzirrhose, welche als Begleiterscheinung bei einer Autoimmunerkrankung auftritt.

38. Verwendung gemäß Anspruch 23, wobei die gastrointestinale Erkrankung aus der Gruppe ausgewählt ist, welche besteht aus: Speiseröhrendysmotilität, entzündlicher Darmerkrankung und Sklerodermie.

39. Verwendung gemäß Anspruch 23, wobei die vaskuläre Erkankung aus der Gruppe ausgewählt ist, welche besteht aus: Atherosklerose oder Reperfusionsverletzung.

40. Verwendung gemäß einem der Ansprüche 23 bis 39, wobei der Aglycosyl-anti-CD154-Antikörper oder das Aglycosyl-anti-CD154-Antikörperderivat aus der Gruppe ausgewählt sind, welche besteht aus: einem monoklonalen Antikörper, einem polyklonalen Antikörper, einem murinen Antikörper, einem chimären Antikörper, einem primatisierten Antikörper, einem humanisierten Antikörper und einem vollständig humanen Antikörper.

41. Verwendung gemäß einem der Ansprüche 23 bis 39, wobei der Aglycosyl-anti-CD154-Antikörper oder das Aglycosyl-anti-CD154-Antikörperderivat aus der Gruppe ausgewählt sind, welche besteht aus: einem multimeren Antikörper, einem heterodimeren Antikörper, einem hemidimeren Antikörper, einem tetravalenten Antikörper und einem bispezifischen Antikörper.

42. Verwendung gemäß einem der Ansprüche 23 bis 39, wobei der Aglycosyl-anti-CD154-Antikörper oder das Aglycosyl-anti-CD154-Antikörperderivat oder die pharmazeutische Zusammensetzung, welche den Antikörper oder das Antikörperderivat umfasst, an den Empfänger verabreicht werden durch Injektion intravenös, subkutan, intraperitoneal, intramuskulär, intramedullär, intraventrikulär, intraepidural, intraarteriell, intravaskulär, intraartikulär, intrasynovial, intrastemal, intrathekal, intrahepatisch, intraspinal, intratumoral, intrakranial; durch einen enteralen, intrapulmonalen, intramukosalen, intrauterinen oder sublingualen Verabreichungsweg; oder lokal an Stellen von Entzündung oder Tumorwachstum.

43. Verwendung gemäß einem der Ansprüche 23 bis 39, wobei der Aglycosyl-anti-CD154-Antikörper, das Aglycosyl-anti-CD154-Antikörperderivat oder die pharmazeutische Zusammensetzung, welche den Antikörper oder das Antikörperderivat umfasst, an den Empfänger über einen Weg verabreicht werden, welcher aus der Gruppe ausgewählt ist, die besteht aus: oralen, nasalen, ophthalmischen, rektalen und topischen Wegen.

44. Verwendung gemäß Anspruch 43, wobei der Aglycosyl-anti-CD154-Antikörper, das Aglycosyl-anti-CD154-Antikörperderivat oder die pharmazeutische Zusammensetzung, welche den Antikörper oder das Antikörperderivat umfasst, an den Empfänger oral in der Form von Kapseln, Tabletten, wässrigen Suspensionen oder Lösungen verabreicht werden.

45. Verwendung gemäß Anspruch 43, wobei der Aglycosyl-anti-CD154-Antikörper, das Aglycosyl-anti-CD154-Antikörperderivat oder die pharmazeutische Zusammensetzung, welche den Antikörper oder das Antikörperderivat umfasst, an den Empfänger topisch durch Aufbringung einer Creme, Salbe oder dergleichen verabreicht werden.

46. Verwendung gemäß Anspruch 43, wobei der Aglycosyl-anti-CD154-Antikörper, das Aglycosyl-anti-CD154-Antikörperderivat oder die pharmazeutische Zusammensetzung, welche den Antikörper oder das Antikörperderivat umfasst, an den Empfänger durch Inhalation durch die Verwendung einer Zerstäubungsvorrichtung, einer Trockenpulverinhalationsvorrichtung oder einer Inhalationsvorrichtung mit festgelegter Dosierung verabreicht werden.

47. Verwendung gemäß einem der Ansprüche 23 bis 39, wobei der Aglycosyl-anti-CD154-Antikörper, das Aglycosyl-anti-CD154-Antikörperderivat oder die pharmazeutische Zusammensetzung, welche den Antikörper oder das Antikörperderivat umfasst, an den Empfänger durch Verabreichung mit verlängerter Freisetzung verabreicht werden.

48. Verwendung gemäß einem der Ansprüche 23 bis 39, wobei der Aglycosyl-anti-CD154-Antikörper, das Aglycosyl-anti-CD154-Antikörperderivat oder die pharmazeutische Zusammensetzung, welche den Antikörper oder das Antikörperderivat umfasst, an den Empfänger zusammen mit einem therapeutischen Mittel verabreicht werden.

49. Verwendung gemäß einem der Ansprüche 23 bis 39, wobei der Aglycosyl-anti-CD154-Antikörper, das Aglycosyl-anti-CD154-Antikörperderivat oder die pharmazeutische Zusammensetzung, welche den Antikörper oder das Antikörperderivat umfasst, an den Empfänger in mehreren Dosen pro Tag verabreicht werden.

50. Verwendung gemäß einem der Ansprüche 23 bis 39, wobei der Aglycosyl-anti-CD154-Antikörper, das Aglycosyl-anti-CD154-Antikörperderivat oder die pharmazeutische Zusammensetzung, welche den Antikörper oder das Antikörperderivat umfasst, an den Empfänger wiederholt in Intervallen in einem Bereich von jeden Tag bis jeden zweiten Monat verabreicht werden.

51. Verwendung gemäß einem der Ansprüche 23 bis 39, wobei der Aglycosyl-anti-CD154-Antikörper, das Aglycosyl-anti-CD154-Antikörperderivat oder die pharmazeutische Zusammensetzung, welche den Antikörper oder das Antikörperderivat umfasst, an den Empfänger in Intervallen für eine Zeit, die so lange ist, wie es medizinisch indiziert ist, in einem Bereich von Tagen oder Wochen bis zur Lebensdauer des Empfängers verabreicht werden.

52. Verwendung gemäß einem der Ansprüche 23 bis 39, wobei der Aglycosyl-anti-CD154-Antikörper, das Aglycosyl-anti-CD154-Antikörperderivat oder die pharmazeutische Zusammensetzung, welche den Antikörper oder das Antikörperderivat umfasst, an den Empfänger mit einer immunomodulatorischen oder immunsuppresiven Verbindung verabreicht werden.

53. Verwendung gemäß Anspruch 52, wobei die immunomodulatorische oder immunsuppressive Verbindung aus der Gruppe ausgewählt ist, welche besteht aus:
(a) einem Mittel, welches die costimulatorische T-Zell-Signalgebung über CD28 unterbricht;
(b) einem Mittel, welches die Calcineurin-Signalgebung unterbricht;
(c) einem Corticosteroid;
(d) einem antiproliferativen Mittel; und
(e) einem Antikörper, welcher spezifisch an ein Protein bindet, das auf der Oberfläche von Immunzellen exprimiert wird, einschließlich aber nicht eingeschränkt auf CD45, CD2, IL2R, CD4, CD8 und RANK FcR, B7, CTLA4, TNF, LTβ und VLA-4.

54. Verwendung gemäß Anspruch 52, wobei die immunsuppressive oder immunomodulatorische Verbindung aus der Gruppe ausgewählt ist, welche besteht aus: Takrolimus, Sirolimus, Mycophenolatmofetil, Mizorubin, Deoxyspergualin, Brequinar-Natrium, Leflunomid, Rapamycin und Azaspiran.

55. Verwendung des Aglycosyl-anti-CD154-Antikörpers oder -Antikörperderivats gemäß einem der Ansprüche 1 bis 5 zur Herstellung einer Zusammensetzung zur Verwendung in einem Verfahren zur Bildgebung von Tumorzellen oder neoplastischen Zellen in einem Empfänger, welche ein Protein exprimieren, das spezifisch durch Aglycosyl hu5c8, welcher durch die Zellinie mit der ATCC-Zugangsnr. PTA-4931 hergestellt wird, erkannt wird, wobei das Verfahren die folgenden Schritte umfasst:
(a) das Verabreichen einer wirksamen Menge der Zusammensetzung an den Empfänger unter Bedingungen, welche die Bildung eines Komplexes zwischen dem Antikörper oder Antikörperderivat und einem Protein auf der Oberfläche von Tumorzellen oder neoplastischen Zellen ermöglichen; und
(b) das Bildgeben eines jedweden gebildeten Antikörper/Protein-Komplexes oder Antikörperderivat/Komplexes, wobei jedwede Tumorzellen oder neoplastischen Zellen in dem Empfänger durch Bildgebung dargestellt werden.

56. Verwendung des Aglycosyl-anti-CD154-Antikörpers oder -Antikörperderivats gemäß einem der Ansprüche 1 bis 5 zur Herstellung einer Zusammensetzung zur Verwendung in einem Verfahren zum Nachweisen der Gegenwart von Tumorzellen oder neoplastischen Zellen in einem Empfänger, welche ein Protein exprimieren, das spezifisch durch Aglycosyl hu5c8, welcher durch die Zelllinie mit der ATCC-Zugangsnr. PTA-4931 hergestellt wird, erkannt wird, wobei das Verfahren die folgenden Schritte umfasst:
(a) das Verabreichen einer wirksamen Menge der Zusammensetzung an den Empfänger unter Bedingungen, welche die Bildung eines Komplexes zwischen dem Antikörper oder Antikörperderivat und dem Protein ermöglichen;
(b) das Clearen von jedwedem nicht gebundenen Mittel zur Bildgebung aus dem Empfänger; und
(c) das Nachweisen der Gegenwart von jedwedem gebildeten Antikörper/Protein-Komplex oder Antikörperderivat/Komplex, wobei die Gegenwart eines solchen Komplexes die Gegenwart von Tumorzellen oder neoplastischen Zellen in dem Empfänger anzeigt.

## Revendications

1. Anticorps ou dérivé d'anticorps anti-CD154 aglycosylé, **caractérisé par** une modification au niveau du site N-lié conservé dans les domaines C_{H}2 de la partie Fc dudit anticorps.

2. Anticorps ou dérivé d'anticorps anti-CD154 aglycosylé selon la revendication 1, dans lequel la modification comprend une mutation dans le site de glycosylation de la chaîne lourde, dans laquelle la mutation empêche une glycosylation au niveau du site.

3. Anticorps ou dérivé d'anticorps anti-CD154 aglycosylé selon la revendication 2, dans lequel la modification comprend une mutation de N298Q (N297 selon la numérotation EU de Kabat).

4. Anticorps ou dérivé d'anticorps anti-CD154 aglycosylé selon la revendication 1, dans lequel la modification comprend l'élimination des glycanes du domaine C_{H}2.

5. Anticorps ou dérivé d'anticorps anti-CD154 aglycosylé selon la revendication 1, dans lequel la modification comprend la prévention contre une glycosylation au niveau du domaine C_{H}2.

6. Anticorps ou dérivé d'anticorps anti-CD154 aglycosylé selon l'une quelconque des revendications 1 à 5, dans lequel ledit anticorps anti-CD154 aglycosylé ou dérivé d'anticorps ne se lie pas à un récepteur effecteur.

7. Anticorps ou dérivé d'anticorps anti-CD154 aglycosylé selon l'une quelconque des revendications 1 à 5, dans lequel ledit anticorps ou dérivé d'anticorps anti-CD154 aglycosylé ne provoque pas de thrombose.

8. Anticorps ou dérivé d'anticorps anti-CD154 aglycosylé selon l'une quelconque des revendications 1 à 5, dans lequel ledit anticorps est choisi dans le groupe constitué par : un anticorps monoclonal, un anticorps polyclonal, un anticorps murin, un anticorps chimérique, un anticorps primatisé, un anticorps humanisé et un anticorps totalement humain.

9. Anticorps ou dérivé d'anticorps anti-CD154 aglycosylé selon l'une quelconque des revendications 1 à 5, dans lequel ledit anticorps est choisi dans le groupe constitué par : un anticorps multimère, un anticorps hétérodimère, un anticorps hémidimérique, un anticorps tétravalent et un anticorps bispécifique.

10. Anticorps ou dérivé d'anticorps anti-CD154 aglycosylé selon l'une quelconque des revendications 1 à 5, dans lequel ledit anticorps est le hu5c8 produit par la lignée cellulaire ayant le numéro de dépôt ATCC PTA-4931.

11. Anticorps ou dérivé d'anticorps anti-CD154 aglycosylé selon l'une quelconque des revendications 1 à 5, dans lequel ledit anticorps ou dérivé d'anticorps est marqué avec un marqueur détectable.

12. Anticorps ou dérivé d'anticorps anti-CD154 aglycosylé selon la revendication 11, dans lequel le marqueur détectable est un isotope radioactif, une enzyme, un colorant ou la biotine.

13. Anticorps ou dérivé d'anticorps anti-CD154 aglycosylé selon l'une quelconque des revendications 1 à 5, dans lequel ledit anticorps ou dérivé d'anticorps est conjugué à un agent thérapeutique.

14. Anticorps ou dérivé d'anticorps anti-CD154 aglycosylé selon la revendication 13, dans lequel ledit agent thérapeutique est un radioisotope, un radionucléide, une toxine, un toxoïde ou un agent chimiothérapeutique.

15. Anticorps ou dérivé d'anticorps anti-CD154 aglycosylé selon l'une quelconque des revendications 1 à 5, dans lequel ledit anticorps ou dérivé d'anticorps est conjugué à un agent d'imagerie.

16. Anticorps ou dérivé d'anticorps anti-CD154 aglycosylé selon la revendication 15, dans lequel l'agent d'imagerie est un fragment de marquage.

17. Anticorps ou dérivé d'anticorps anti-CD154 aglycosylé selon la revendication 15, dans lequel l'agent d'imagerie est une biotine, un fragment fluorescent, un fragment radioactif, une étiquette histidine ou une étiquette peptidique.

18. Composition pharmaceutique comprenant l'anticorps ou dérivé d'anticorps anti-CD154 aglycosylé selon l'une quelconque des revendications 1 à 5.

19. Composition pharmaceutique selon la revendication 18, comprenant en outre un support pharmaceutiquement acceptable.

20. Composition pharmaceutique selon la revendication 18, comprenant en outre un composé immunosuppresseur ou immunomodulateur.

21. Lignée cellulaire produisant l'anticorps ou dérivé d'anticorps anti-CD154 aglycosylé selon l'une quelconque des revendications 1 à 5.

22. Lignée cellulaire selon la revendication 21, dans laquelle la lignée cellulaire produit le hu5c8 aglycosylé (numéro de dépôt ATCC PTA-4931).

23. Utilisation d'un anticorps anti-CD154 aglycosylé ou d'un dérivé d'anticorps anti-CD154 aglycosylé selon l'une quelconque des revendications 1 à 5, ou d'une composition pharmaceutique comprenant ledit anticorps ou dérivé d'anticorps, pour la fabrication d'un médicament destiné à inhiber une réponse immunitaire, une réponse inflammatoire, un rejet de greffe, la réaction de greffe contre hôte, une réponse allergique, une réponse auto-immune, une fibrose, une infection virale des lymphocytes T par le virus HTLV I, une maladie gastro-intestinale, une maladie vasculaire ou la prolifération de cellules cancéreuses des lymphocytes T, chez un sujet.

24. Utilisation selon la revendication 23, dans laquelle l'anticorps anti-CD154 aglycosylé, le dérivé d'anticorps ou la composition pharmaceutique inhibe la liaison du CD154 au CD40.

25. Utilisation selon la revendication 23, dans laquelle l'anticorps anti-CD154 aglycosylé, le dérivé d'anticorps ou la composition pharmaceutique est capable de se lier spécifiquement à une protéine qui est reconnue spécifiquement par le hu5c8 aglycosylé, qui est produit par la lignée cellulaire ayant leur numéro de dépôt ATCC PTA-4931.

26. Utilisation selon la revendication 23, dans laquelle l'anticorps anti-CD154 aglycosylé, le dérivé d'anticorps ou la composition pharmaceutique se lie spécifiquement à l'épitope auquel le hu5c8 aglycosylé (numéro de dépôt ATCC PTA-4931) se lie spécifiquement.

27. Utilisation selon la revendication 23, dans laquelle la réponse inflammatoire est choisie dans le groupe constitué par : l'arthrite, la dermatite de contact, l'hypergammaglobulinémie IgE, l'affection abdominale inflammatoire, l'asthme allergique et une maladie inflammatoire idiopathique.

28. Utilisation selon la revendication 27, dans laquelle l'arthrite est choisie dans le groupe constitué par : la polyarthrite rhumatoïde, une arthrite inflammatoire non rhumatoïde, une arthrite associée à la maladie de Lyme et une arthrose inflammatoire.

29. Utilisation selon la revendication 27, dans laquelle la maladie inflammatoire idiopathique est choisie dans le groupe constitué par : le psoriasis et le lupus systémique.

30. Utilisation selon la revendication 23, dans laquelle le rejet de greffe implique la transplantation d'un coeur, d'un rein, d'un foie, de peau, des îlots de Langerhans du pancréas ou de moelle osseuse.

31. Utilisation selon la revendication 23, dans laquelle la réponse allergique est choisie dans le groupe constitué par : le rhume des foins ou une allergie à la pénicilline ou à d'autres médicaments.

32. Utilisation selon la revendication 23, dans laquelle la réponse auto-immune dérive d'une maladie infectieuse.

33. Utilisation selon la revendication 23, dans laquelle la réponse auto-immune dérive du syndrome de Reiter, d'une spondylarthrite, de la maladie de Lyme, des infections par le VIH, de la syphilis ou de la tuberculose.

34. Utilisation selon la revendication 23, dans laquelle la réponse auto-immune est choisie dans le groupe constitué par : la polyarthrite rhumatoïde, la myasthénie gravis, le lupus érythémateux disséminé, la maladie de Graves, le purpura thrombopénique idiopathique, l'anémie hémolytique, le diabète sucré, l'affection abdominale inflammatoire, la maladie de Crohn, la sclérose en plaques, le psoriasis, les maladies auto-immunes induites par des médicaments et le lupus induit par les médicaments.

35. Utilisation selon la revendication 23, dans laquelle la fibrose est choisie dans le groupe constitué par : la fibrose pulmonaire et une maladie fibrotique.

36. Utilisation selon la revendication 35, dans laquelle la fibrose pulmonaire est choisie dans le groupe constitué par : une fibrose pulmonaire faisant suite à un syndrome de détresse respiratoire de l'adulte, une fibrose pulmonaire induite par les médicaments, une fibrose pulmonaire idiopathique et une pneumonie par hypersensibilité.

37. Utilisation selon la revendication 35, dans laquelle la maladie fibrotique est choisie dans le groupe constitué par : l'hépatite C ; l'hépatite B ; la cirrhose ; une cirrhose du foie faisant suite à une attaque toxique ; une cirrhose du foie induite par les médicaments ; une cirrhose du foie faisant suite à une infection virale et une cirrhose du foie faisant suite à une maladie auto-immune.

38. Utilisation selon la revendication 23, dans laquelle la maladie gastro-intestinale est choisie dans le groupe constitué par : la dysmotilité oesophagienne, l'affection abdominale inflammatoire et la sclérodermie.

39. Utilisation selon la revendication 23, dans laquelle la maladie vasculaire est choisie dans le groupe constitué par : l'athérosclérose et la lésion de reperfusion.

40. Utilisation selon l'une quelconque des revendications 23 à 39, dans laquelle l'anticorps anti-CD154 aglycosylé ou le dérivé d'anticorps anti-CD154 aglycosylé est choisi dans le groupe constitué par : un anticorps monoclonal, un anticorps polyclonal, un anticorps murin, un anticorps chimérique, un anticorps primatisé, un anticorps humanisé et un anticorps totalement humain.

41. Utilisation selon l'une quelconque des revendications 23 à 39, dans laquelle l'anticorps anti-CD154 aglycosylé ou le dérivé d'anticorps anti-CD154 aglycosylé est choisi dans le groupe constitué par : un anticorps multimère, un anticorps hétérodimère, un anticorps hémidimérique, un anticorps tétravalent et un anticorps bispécifique.

42. Utilisation selon l'une quelconque des revendications 23 à 39, dans laquelle l'anticorps anti-CD154 aglycosylé ou le dérivé d'anticorps anti-CD154 aglycosylé ou la composition pharmaceutique contenant ledit anticorps ou dérivé d'anticorps doit être administré audit sujet par injection intraveineuse, sous-cutanée, intrapéritonéale, intramusculaire, intramédullaire, intraventriculaire, intraépidurale, intraartérielle, intravasculaire, intraarticulaire, intrasynoviale, intrastemale, intrathécale, intrahépatique, intraspinale, intratumorale, intracrânienne ; ou par une voie d'administration entérale, intrapulmonaire, transmucosale, intrautérine ou sublinguale ; ou localement au niveau des sites d'inflammation ou de la croissance de la tumeur.

43. Utilisation selon l'une quelconque des revendications 23 à 39, dans laquelle l'anticorps anti-CD154 aglycosylé, le dérivé d'anticorps anti-CD154 aglycosylé ou la composition pharmaceutique comprenant ledit anticorps ou dérivé d'anticorps doit être administré audit sujet par une voie choisie dans le groupe constitué par : la voie orale, la voie nasale, la voie ophtalmique, la voie rectale et la voie topique.

44. Utilisation selon la revendication 43, dans laquelle l'anticorps anti-CD154 aglycosylé, le dérivé d'anticorps anti-CD154 aglycosylé ou la composition pharmaceutique comprenant ledit anticorps ou dérivé d'anticorps doit être administré audit sujet par voie orale sous la forme de capsules, de comprimés, de suspensions ou solutions aqueuses.

45. Utilisation selon la revendication 43, dans laquelle l'anticorps anti-CD154 aglycosylé, le dérivé d'anticorps anti-CD154 aglycosylé ou la composition pharmaceutique comprenant ledit anticorps ou dérivé d'anticorps doit être administré audit sujet par voie topique par l'application d'une crème, d'un onguent ou équivalents.

46. Utilisation selon la revendication 43, dans laquelle l'anticorps anti-CD154 aglycosylé, le dérivé d'anticorps anti-CD154 aglycosylé ou la composition pharmaceutique comprenant ledit anticorps ou dérivé d'anticorps doit être administré audit sujet par inhalation au moyen d'un nébuliseur, d'un inhalateur de poudre sèche ou d'un inhalateur de dose.

47. Utilisation selon l'une quelconque des revendications 23 à 39, dans laquelle l'anticorps anti-CD154 aglycosylé, le dérivé d'anticorps anti-CD154 aglycosylé ou la composition pharmaceutique comprenant ledit anticorps ou dérivé d'anticorps doit être administré audit sujet par une administration à libération prolongée.

48. Utilisation selon l'une quelconque des revendications 23 à 39, dans laquelle l'anticorps anti-CD154 aglycosylé, le dérivé d'anticorps anti-CD154 aglycosylé ou la composition pharmaceutique comprenant ledit anticorps ou dérivé d'anticorps doit être administré audit sujet avec un agent thérapeutique.

49. Utilisation selon l'une quelconque des revendications 23 à 39, dans laquelle l'anticorps anti-CD154 aglycosylé, le dérivé d'anticorps anti-CD154 aglycosylé ou la composition pharmaceutique comprenant ledit anticorps ou dérivé d'anticorps doit être administré audit sujet en de multiples doses par jour.

50. Utilisation selon l'une quelconque des revendications 23 à 39, dans laquelle l'anticorps anti-CD154 aglycosylé, le dérivé d'anticorps anti-CD154 aglycosylé ou la composition pharmaceutique comprenant ledit anticorps ou dérivé d'anticorps doit être administré audit sujet à plusieurs reprises à des intervalles allant de chaque jour à tous les autres mois.

51. Utilisation selon l'une quelconque des revendications 23 à 39, dans laquelle l'anticorps anti-CD154 aglycosylé, le dérivé d'anticorps anti-CD154 aglycosylé ou la composition pharmaceutique comprenant ledit anticorps ou dérivé d'anticorps doit être administré audit sujet à des intervalles pendant une durée où cela est médicalement indiqué, allant de quelques jours ou quelques semaines à la vie entière du sujet.

52. Utilisation selon l'une quelconque des revendications 23 à 39, dans laquelle l'anticorps anti-CD154 aglycosylé, le dérivé d'anticorps anti-CD154 aglycosylé ou la composition pharmaceutique comprenant ledit anticorps ou dérivé d'anticorps doit être administré audit sujet un composé immunosuppresseur ou immunomodulateur.

53. Utilisation selon la revendication 52, dans laquelle le composé immunosuppresseur ou immunomodulateur est choisi dans le groupe constitué par :
(a) un agent qui interrompt la signalisation de costimulation des lymphocytes T *via* CD28 ;
(b) un agent qui interrompt la signalisation de la calcineurine,
(c) un corticostéroïde,
(d) un agent anti-prolifératif ; et
(e) un anticorps se liant spécifiquement à une protéine exprimée sur la surface des cellules immunitaires telles que, mais sans s'y limiter, CD45, CD2, IL2R, CD4, CD8 et RANK FcR, B7, CTLA4, TNF, LTβ et VLA-4.

54. Utilisation selon la revendication 52, dans laquelle le composé immunosuppresseur ou immunomodulateur est choisi dans le groupe constitué par :
le tacrolimus, le sirolimus, le mycophénolate mofétil, la mizorubine, la désoxyspergualine, le bréquinar sodique, le léflunomide, la rapamycine et
l'azaspirane.

55. Utilisation d'un anticorps ou dérivé d'anticorps anti-CD154 aglycosylé selon l'une quelconque des revendications 1 à 5, pour la fabrication d'une composition utilisable dans un procédé d'imagerie de cellules tumorales ou de cellules néoplasiques chez un sujet qui exprime une protéine qui est spécifiquement reconnue par le hu5c8 aglycosylé produit par la lignée cellulaire ayant le numéro de dépôt ATCC PTA-4931, ledit procédé comprenant les étapes suivantes :
(a) l'administration au sujet d'une quantité efficace de la composition dans des conditions permettant la formation d'un complexe entre l'anticorps ou dérivé anticorps et une protéine sur la surface de cellules tumorales ou de cellules néoplasiques ; et
(b) la formation d'une image à partir de n'importe quel complexe anticorps/protéine ou dérivé d'anticorps/complexe formé, pour ainsi obtenir une image des cellules tumorales ou des cellules néoplasiques chez le sujet.

56. Utilisation d'un anticorps ou dérivé d'anticorps anti-CD154 aglycosylé selon l'une quelconque des revendications 1 à 5, pour la fabrication d'une composition utilisable dans un procédé de détection de la présence de cellules tumorales ou de cellules néoplasiques chez un sujet qui expriment une protéine qui est spécifiquement reconnue par le hu5c8 aglycosylé produit par la lignée cellulaire ayant le numéro de dépôt ATCC PTA-4931, ledit procédé comprenant les étapes suivantes :
(a) l'administration au sujet d'une quantité efficace de la composition dans des conditions permettant la formation d'un complexe entre l'anticorps ou dérivé anticorps et la protéine ;
(b) l'élimination des agents d'imagerie non liés du sujet ; et
(c) la détection de la présence d'un quelconque complexe anticorps/protéine ou dérivé d'anticorps/complexe formé, la présence d'un tel complexe indiquant la présence de cellules tumorales ou de cellules néoplasiques chez le sujet.
